(19) Europäisches Patentamt
European Patent Office
Office européen des brevets

(11) **EP 3 649 939 B1**

(12) **EUROPEAN PATENT SPECIFICATION**

(45) Date of publication and mention
of the grant of the patent:
**12.05.2021 Bulletin 2021/19**

(21) Application number: **17916527.9**

(22) Date of filing: **04.07.2017**

(51) Int Cl.:
**A61B 5/11** *(2006.01)* **A61B 5/00** *(2006.01)*

(86) International application number:
**PCT/JP2017/024560**

(87) International publication number:
**WO 2019/008688 (10.01.2019 Gazette 2019/02)**

(54) **INFORMATION PROCESSING DEVICE, INFORMATION PROCESSING SYSTEM, AND INFORMATION PROCESSING METHOD**

INFORMATIONSVERARBEITUNGSVORRICHTUNG, INFORMATIONSVERARBEITUNGSSYSTEM UND INFORMATIONSVERARBEITUNGSVERFAHREN

DISPOSITIF DE TRAITEMENT D'INFORMATIONS, SYSTÈME DE TRAITEMENT D'INFORMATIONS ET PROCÉDÉ DE TRAITEMENT D'INFORMATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.05.2020 Bulletin 2020/20**

(73) Proprietor: **Fujitsu Limited**
**Kawasaki-shi, Kanagawa 211-8588 (JP)**

(72) Inventors:
• **HOTTA, Shinji**
**Kawasaki-shi**
**Kanagawa 211-8588 (JP)**
• **INOMATA, Akihiro**
**Kawasaki-shi**
**Kanagawa 211-8588 (JP)**

(74) Representative: **Haseltine Lake Kempner LLP**
**138 Cheapside**
**London EC2V 6BJ (GB)**

(56) References cited:
WO-A1-2017/109920   CN-A- 106 908 021
JP-A- 2010 121 994   JP-A- 2012 179 114
JP-A- 2016 137 228   JP-A- 2016 158 887
JP-A- 2016 214 348   JP-A- 2016 220 922
US-A1- 2016 100 776

• BAGALCIAGUE F ET AL: "Unstable gait assessment with a portable analysis system", 2014 IEEE INTERNATIONAL INSTRUMENTATION AND MEASUREMENT TECHNOLOGY CONFERENCE (I2MTC) PROCEEDINGS, IEEE, 12 May 2014 (2014-05-12), pages 181-185, XP032620852, DOI: 10.1109/I2MTC.2014.6860729 [retrieved on 2014-07-18]

EP 3 649 939 B1

**Description**

TECHNICAL FIELD

The present invention relates to an information processing apparatus, an information processing system, and an information processing method.

BACKGROUND ART

In a medical field, it has been conventionally required to grasp a condition of a patient in order to provide appropriate medical treatment for the patient. For example, the way of walking of a patient tends to be influenced by the degree of physical disorder due to a disease, the degree of recovery of a disease or injury, and the like, and, in some cases, it is required to grasp the way of walking of the patient in order to grasp a condition of the patient. Therefore, it is required to accurately calculate feature values of the way of walking of the patient, such as a step length and a point of time at which each leg lands, and grasp the way of walking of the patient on the basis of the feature values. In particular, it is preferable to grasp the daily way of walking of the patient in order to provide appropriate medical treatment for the patient.

In a related art, for example, the number of steps and a walking cycle obtained when a walker walks are detected on the basis of vertical acceleration obtained by a three-axis acceleration sensor, and a travel direction acceleration per step is measured by the acceleration sensor, and then a step length is estimated on the basis of the travel direction acceleration and the walking cycle. Furthermore, in another related art, a point of time at which each leg lands is estimated by gyro sensors attached to both the legs, and a change in angle between the points of time of landing is measured, and then a step length is estimated on the basis of the change in angle.

Further, for example, there is a technique in which, based on absolute values of acceleration of detection data acquired from walking sensors attached to legs of a walker, a timing of a maximum value in a former part of the data of each step is calculated as a separation time and a timing of a maximum value in a latter part thereof is calculated as a contact time. Still further, for example, there is a technique of measuring both-legs contact time period on the basis of sensor output from a sensor that is attached to a human body and measures acceleration or pressure applied to the human body, the both-legs contact time period being a time period from landing of a heel of one leg to separation of a toe of the other leg during walking.

CITATION LIST

PATENT DOCUMENT

Patent Document 1: Japanese Laid-open Patent Publication No. 2004-163168
Patent Document 2: Japanese Laid-open Patent Publication No. 2008-175559
Patent Document 3: Japanese Laid-open Patent Publication No. 2012-179114
Patent Document 4: Japanese Laid-open Patent Publication No. 2002-197437

NON-PATENT DOCUMENT

Non-Patent Document 1: Wu, Xiaoxu, Yan Wang, and Gregory Pottie, "A robust step length estimation system for human gait using motion sensors." Proceedings of the conference on Wireless Health. ACM, 2015. Reference may be made to any of: a paper by BAGALCIAGUE F ET AL, titled "Unstable gait assessment with a portable analysis system", submitted in 2014 IEEE INTERNATIONAL INSTRUMENTATION AND MEASUREMENT TECHNOLOGY CONFERENCE (I2MTC) PROCEEDINGS, 12 May 2014 (2014-05-12), pages 181-185, XP032620852, DOI:10.1109/12MTC.2814.6860729; and US 2016/100776 A1, which is related to fall detection and fall risk detection systems and methods, in particular to a light-weight, small and portable ambulatory sensor for measuring and monitoring a person's physical activity, wherein based on these measurements and computations the system quantifies the subject's physical activity, quantifies the subject's gait, determines his or her risk of falling, and automatically detects falls, wherein high autonomy is achieved by using only accelerometers, which have low power consumption rates as compared with gyroscope-based systems, wherein accelerometer measurements however, contain significant amounts of noise, which must be removed before further analysis.

SUMMARY OF INVENTION

TECHNICAL PROBLEM

In some cases, it is desired to attach a sensor device to a patient, estimate a point of time at which each leg of the patient lands on the basis of time series data obtained from the sensor device, and estimate a step length of the patient on the basis of the estimated point of time. However, when the way of walking changes depending on the patient, a place where the patient walks, a surrounding environment in which the patient walks, a condition of the patient, or the like, it tends to be difficult to accurately specify the point of time at which each leg of the patient lands. For example, in a case where the patient walks at a low speed or other cases, changes in acceleration and angular velocity caused by landing are small. This makes it difficult to uniquely specify the point of time at which each leg of the patient lands. Further, this makes it difficult to accurately estimate the step length.

In one aspect, it is an object of the present invention to provide an information processing apparatus, an information processing system, and an information processing method capable of improving accuracy of specifying a point of time corresponding to landing of each leq of a subject.

SOLUTION TO PROBLEM

According to an embodiment, there is proposed an information processing apparatus, an information processing system, an information processing method, each of which acquires measurement information regarding movement of each leg of a subject, specifies, for each step in a walking period of the subject, one or more candidates for a point of time corresponding to landing of a leg serving as a swing leg of the subject on the basis of the acquired measurement information, specifies a plurality of candidate groups obtained by selecting one of the one or more candidates specified for each step in the walking period and combining the selected candidates, calculates, for each candidate group of the plurality of specified candidate groups, a step length of each step in the walking period and calculates, for each step in the walking period, a cumulative travel distance of the leg serving as the swing leg of the subject on the basis of the calculated step length of each step in the walking period, evaluates, for each candidate group, a degree of appearance of a relationship on the basis of the cumulative travel distance calculated for each step in the walking period, the relationship being a relationship in which a landing position of the leg serving as the swing leg of the subject falls within a predetermined range of a landing position of a leg serving as the support leg of the subject in the walking period, selects one of the plurality of candidate groups on the basis of a result of the evaluation, and outputs the selected one of the plurality of candidate groups.

ADVANTAGEOUS EFFECTS OF INVENTION

According to an aspect of the present invention, it is possible to improve accuracy of specifying a point of time corresponding to landing of each leg of a subject.

BRIEF DESCRIPTION OF DRAWINGS

FIG. 1 is an explanatory diagram illustrating an example of an information processing method according to an embodiment.
FIG. 2 is an explanatory diagram illustrating an example of an information processing system 200.
FIG. 3 is a block diagram illustrating an exemplary hardware configuration of an information processing apparatus 100.
FIG. 4 is a block diagram illustrating an exemplary hardware configuration of a measuring instrument 201.
FIG. 5 is a block diagram illustrating an exemplary functional configuration of the information processing apparatus 100.
FIG. 6 is an explanatory diagram illustrating an example of a flow in which the information processing apparatus 100 selects a candidate group.
FIG. 7 is a first explanatory diagram illustrating a specific example in which the information processing apparatus 100 selects a candidate group.
FIG. 8 is a second explanatory diagram illustrating a specific example in which the information processing apparatus 100 selects a candidate group.
FIG. 9 is a third explanatory diagram illustrating a specific example in which the information processing apparatus 100 selects a candidate group.

FIG. 10 is a fourth explanatory diagram illustrating a specific example in which the information processing apparatus 100 selects a candidate group.

FIG. 11 is a fifth explanatory diagram illustrating a specific example in which the information processing apparatus 100 selects a candidate group.

FIG. 12 is a sixth explanatory diagram illustrating a specific example in which the information processing apparatus 100 selects a candidate group.

FIG. 13 is a seventh explanatory diagram illustrating a specific example in which the information processing apparatus 100 selects a candidate group.

FIG. 14 is an eighth explanatory diagram illustrating a specific example in which the information processing apparatus 100 selects a candidate group.

FIG. 15 is a ninth explanatory diagram illustrating a specific example in which the information processing apparatus 100 selects a candidate group.

FIG. 16 is a tenth explanatory diagram illustrating a specific example in which the information processing apparatus 100 selects a candidate group.

FIG. 17 is an eleventh explanatory diagram illustrating a specific example in which the information processing apparatus 100 selects a candidate group.

FIG. 18 is a twelfth explanatory diagram illustrating a specific example in which the information processing apparatus 100 selects a candidate group.

FIG. 19 is a thirteenth explanatory diagram illustrating a specific example in which the information processing apparatus 100 selects a candidate group.

FIG. 20 is a fourteenth explanatory diagram illustrating a specific example in which the information processing apparatus 100 selects a candidate group.

FIG. 21 is a fifteenth explanatory diagram illustrating a specific example in which the information processing apparatus 100 selects a candidate group.

FIG. 22 is a sixteenth explanatory diagram illustrating a specific example in which the information processing apparatus 100 selects a candidate group.

FIG. 23 is a seventeenth explanatory diagram illustrating a specific example in which the information processing apparatus 100 selects a candidate group.

FIG. 24 is an eighteenth explanatory diagram illustrating a specific example in which the information processing apparatus 100 selects a candidate group.

FIG. 25 is a nineteenth explanatory diagram illustrating a specific example in which the information processing apparatus 100 selects a candidate group.

FIG. 26 is a twentieth explanatory diagram illustrating a specific example in which the information processing apparatus 100 selects a candidate group.

FIG. 27 is a twenty-first explanatory diagram illustrating a specific example in which the information processing apparatus 100 selects a candidate group.

FIG. 28 is a twenty-second explanatory diagram illustrating a specific example in which the information processing apparatus 100 selects a candidate group.

FIG. 29 is a twenty-third explanatory diagram illustrating a specific example in which the information processing apparatus 100 selects a candidate group.

FIG. 30 is a twenty-fourth explanatory diagram illustrating a specific example in which the information processing apparatus 100 selects a candidate group.

FIG. 31 is a twenty-fifth explanatory diagram illustrating a specific example in which the information processing apparatus 100 selects a candidate group.

FIG. 32 is a twenty-sixth explanatory diagram illustrating a specific example in which the information processing apparatus 100 selects a candidate group.

FIG. 33 is a twenty-seventh explanatory diagram illustrating a specific example in which the information processing apparatus 100 selects a candidate group.

FIG. 34 is a twenty-eighth explanatory diagram illustrating a specific example in which the information processing apparatus 100 selects a candidate group.

FIG. 35 is a twenty-ninth explanatory diagram illustrating a specific example in which the information processing apparatus 100 selects a candidate group.

FIG. 36 is a thirtieth explanatory diagram illustrating a specific example in which the information processing apparatus 100 selects a candidate group.

FIG. 37 (37A and 37B) is a flowchart illustrating an example of an overall processing procedure.

FIG. 38 is a flowchart illustrating an example of a final certainty factor calculation processing procedure.

FIG. 39 is a flowchart illustrating an example of a first certainty factor calculation processing procedure.

DESCRIPTION OF EMBODIMENTS

Hereinafter, embodiments of an information processing apparatus, an information processing system, and an information processing method according to the present invention will be described in detail with reference to the drawings.

(One Example of Information Processing Method According to Embodiment)

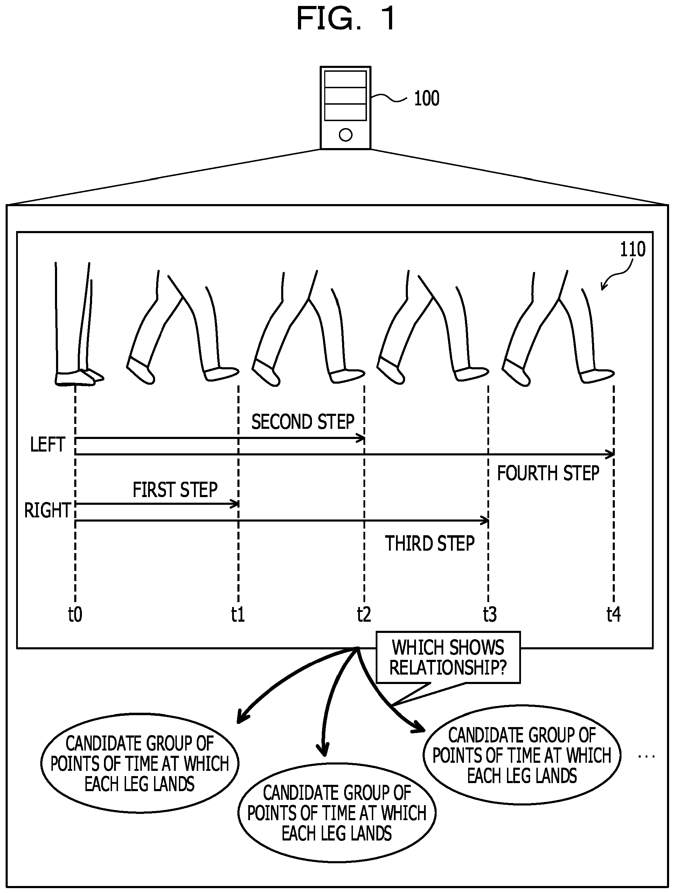

FIG. 1 is an explanatory diagram illustrating an example of an information processing method according to an embodiment. An information processing apparatus 100 is a computer that specifies a point of time at which each leg of a subject lands. The subject is a living body. The subject is, for example, a human. For example, the subject is a subject to be examined by a medical institution. Specifically, the subject is a patient who receives diagnosis, medical care, follow-up, health management, or the like from a medical person such as a medical doctor.

Further, the subject may specifically be a patient who receives rehabilitation instruction or the like from a medical person such as a medical doctor. Furthermore, the subject may specifically be a trainee who receives exercise instruction from a sports instructor. The subject may be, for example, an individual who performs his/her own health management. The subject may be an animal. In the following description, a case where the subject is a "patient" will be mainly described.

Herein, as described above, in a medical field, it is required to grasp a condition of a patient in order to provide appropriate medical treatment for the patient. For example, the way of walking of a patient tends to be influenced by the degree of physical disorder due to a disease, the degree of recovery of a disease or injury, and the like, and, in some cases, it is required to grasp the way of walking of the patient in order to grasp a condition of the patient. Therefore, it is required to accurately calculate a feature value regarding the way of walking of the patient, such as a step length of the patient, and grasp the way of walking of the patient on the basis of the feature value. In particular, it is preferable to grasp the daily way of walking of the patient in order to provide appropriate medical treatment for the patient.

Regarding this, for example, the following case is considered: a point of time at which each leg of the patient lands is specified on the basis of sensor data in which acceleration of each leg of the patient measured by a sensor device attached to the patient is arranged in time series, and the feature value such as a step length of the patient is calculated on the basis of those points of time. However, in this case, it may be difficult to accurately calculate the feature value such as a step length of the patient. For example, changes in a place where the patient walks, a surrounding environment in which the patient walks, a condition of the patient, and the like make it difficult to accurately specify the point of time at which each leg of the patient lands. This makes it difficult to accurately calculate the step length of the patient.

Meanwhile, for example, the following case is considered: sensor devices are attached to various parts of lower limbs of the patient in order to improve accuracy of specifying the point of time at which each leg of the patient lands and to improve accuracy of measuring the feature value such as a step length of the patient. However, this increases a burden on the patient. For example, when the patient wears a sensor device on his/her thigh, the patient tends to have difficulty in walking. In addition, the patient may feel a burden and stress and may lose his/her motivation to use a sensor device in order to receive diagnosis, medical care, follow-up, health management, or the like for the patient.

Therefore, in order to specify the point of time at which each leg of the patient lands and measure the feature value such as a step length of the patient on a daily basis, the number of sensor devices attached to the patient is preferably relatively small, and a part to which the sensor device is attached is preferably a part where the patient feels relatively less burdensome and stressed. Specifically, it is preferable not to attach the sensor device to the thigh of the patient but to attach the sensor device only to a shin of the patient.

Therefore, in this embodiment, there will be described an information processing method in which one or more candidates for the point of time at which each leg of the patient lands are specified once and which one of various combinations of the candidates is likely to be a combination of the points of time at which each leg of the patient lands can be determined. According to this, this embodiment can specify a likely combination of candidates of the points of time at which each leg of the patient lands and can accurately calculate the feature value such as a step length of the patient.

In FIG. 1, the information processing apparatus 100 acquires measurement information regarding movement of each leg of the patient. The measurement information is, for example, time-series data in which angular velocity and acceleration measured by an angular velocity sensor and an acceleration sensor of a measuring instrument attached to each leg of the patient are arranged in time series. The time-series data is, for example, sensor data.

The information processing apparatus 100 receives, for example, sensor data of angular velocity and acceleration of a shin of a left leg of the patient from the measuring instrument attached to the shin of the left leg of the patient. Further, the information processing apparatus 100 receives sensor data of angular velocity and acceleration of a shin of a right leg of the patient from the measuring instrument attached to the shin of the right leg of the patient. With this, the information processing apparatus 100 can use the sensor data to specify the step length of the patient.

Then, based on the acquired measurement information, the information processing apparatus 100 specifies, for each step in a walking period of the patient, one or more candidates for the point of time corresponding to landing of a leg serving as a swing leg of the patient. One step in the walking period is movement in which the patient separates his/her leg from the ground, swings the leg, and lands the leg.

In the following description, the movement of one step is also referred to as "step". In the following description, separating the leg from the ground is also referred to as "toe-off". In the following description, landing the leg is also referred to as "heel-strike".

Then, the information processing apparatus 100 specifies a plurality of candidate groups obtained by selecting one of the one or more candidates specified for each step in the walking period and combining the selected candidates.

Then, the information processing apparatus 100 calculates the step length of each step in the walking period for each of the plurality of specified candidate groups, and, based on the calculated step length of each step in the walking period, calculates a cumulative travel distance of the leg serving as the swing leg of the patient for each step in the walking period. The swing leg is a leg to which the weight of the patient is not applied and is a leg that steps.

For example, the right leg is the swing leg in the first step, and thus the information processing apparatus 100 sets a step length of the right leg in the first step as a cumulative travel distance of the right leg. For example, the left leg is the swing leg in the second step, and thus the information processing apparatus 100 sets a step length of the left leg in the second step as a cumulative travel distance of the left leg. For example, the right leg is the swing leg in the third step, and thus the information processing apparatus 100 adds a step length of the right leg in the third step to the cumulative travel distance of the right leg. For example, the left leg is the swing leg in the fourth step, and thus the information processing apparatus 100 adds a step length of the left leg in the fourth step to the cumulative travel distance of the left leg.

Herein, walking movement of the patient has such a property that a landing position of the leg serving as the swing leg falls within a predetermined range of a landing position of a leg serving as a support leg, as illustrated in a schematic diagram 110 of walking movement of the patient. The support leg is a leg that supports the weight of the patient during a step.

In the example of FIG. 1, at a point of time t1, the landing position of the right leg serving as the swing leg in the first step falls within a predetermined range in front of the landing position of the left leg serving as the support leg. The landing position of the left leg serving as the support leg is a landing position at the start of walking movement. Similarly, at a point of time t2, the landing position of the left leg serving as the swing leg in the second step falls within a predetermined range in front of the landing position of the right leg serving as the support leg. The landing position of the right leg serving as the support leg can be specified on the basis of the landing position of the right leg in the first step at the point of time t1 at which the right leg has previously been the swing leg. Similarly, at a point of time t3, the landing position of the right leg serving as the swing leg in the third step falls within a predetermined range in front of the landing position of the left leg serving as the support leg. Similarly, at a point of time t4, the landing position of the left leg serving as the swing leg in the fourth step falls within a predetermined range in front of the landing position of the right leg serving as the support leg.

Hereinafter, the information processing apparatus 100 uses this property to evaluate, for each candidate group, likelihood of each candidate group obtained by combining the points of time at which each leg lands.

Based on the cumulative travel distance calculated for each step in the walking period, the information processing apparatus 100 evaluates, for each candidate group, a degree of appearance of a predetermined relationship between the landing positions of both the legs of the patient in the walking period. The predetermined relationship is a relationship in which the landing position of the leg serving as the swing leg of the patient falls within a predetermined range of the landing position of the leg serving as the support leg of the patient.

Herein, for example, the patient tends to walk while moving the swing leg ahead of the support leg. Therefore, the information processing apparatus 100 preferably uses, as the predetermined relationship, a relationship in which the landing position of the leg serving as the swing leg of the patient falls within a predetermined range in front of the landing position of the leg serving as the support leg of the patient.

Meanwhile, for example, the patient may drag his/her leg due to illness or injury, and the patient may walk while moving the swing leg to a position existing at a predetermined distance behind the support leg. Therefore, when considering disease or injury, the information processing apparatus 100 may use, as the predetermined relationship, a relationship in which the landing position of the leg serving as the swing leg of the patient falls within a predetermined range in front of a start point existing at a predetermined distance behind the landing position of the leg serving as the support leg of the patient.

The information processing apparatus 100 selects one of the plurality of candidate groups on the basis of the evaluation result and outputs the selected one of the candidate groups. For example, as a result of evaluation, in a case where there is a candidate group in which the predetermined relationship appears in all the steps in the walking period, the information processing apparatus 100 selects and outputs the candidate group.

With this, the information processing apparatus 100 can output a likely candidate group of a combination of the points of time at which each leg of the patient lands and can accurately calculate the step length of the patient on the basis of the candidate group.

Further, based on the output candidate group, the information processing apparatus 100 can accurately calculate not only the step length of the patient but also a predetermined feature value regarding the way of walking of the patient which can be calculated on the basis of the candidate group. The predetermined feature value regarding the way of walking of the patient is, for example, angular velocity, acceleration, or the like of the leg of the patient in a candidate of the point of time at which the patient lands and indicates whether or not the patient walks fast, whether or not the leg of the patient lands slowly, or the like.

The information processing apparatus 100 can accurately calculate the step length of the patient even in a case where sensor devices are not attached to various parts of the lower limbs of the patient. For example, in a case where the sensor device is attached to the shin of the patient where the patient is relatively less burdensome and stressed when the patient wears the sensor device, the information processing apparatus 100 can accurately calculate the step length of the patient.

The number of sensor devices to be attached may be small, and thus the patient has less difficulty in walking and is less burdensome and stressed. Then, this prevents the patient from losing his/her motivation to use the sensor device in order to receive diagnosis, medical care, follow-up, health management, or the like. As a result, the patient can easily use the sensor device on a daily basis, and the information processing apparatus 100 can calculate the daily step length of the patient.

Further, by outputting the step length of the patient, the information processing apparatus 100 can notify a medical person such as a medical doctor of the step length of the patient. Therefore, the information processing apparatus 100 can make it easier for the medical person such as a medical doctor to grasp the way of walking of the patient, and can allow the medical person to efficiently perform diagnosis, medical care, follow-up, health management, or the like for the patient.

The medical person such as a medical doctor can refer to the step length of the patient, grasp the way of walking of the patient, and efficiently perform diagnosis, medical care, follow-up, health management, or the like for the patient. For example, the medical person such as a medical doctor can refer to the daily step length of the patient and grasp the daily way of walking of the patient.

Further, the information processing apparatus 100 can reduce the number of sensor devices to be attached to the patient. Therefore, the information processing apparatus 100 can reduce a cost of using the sensor device in performing diagnosis, medical care, follow-up, health care, or the like for the patient.

(One Example of Information Processing System 200)

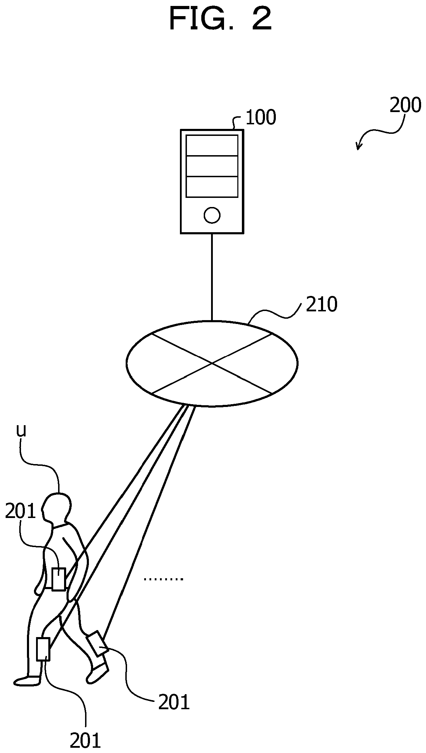

Next, an example of an information processing system 200 to which the information processing apparatus 100 illustrated in FIG. 1 is applied will be described with reference to FIG. 2.

FIG. 2 is an explanatory diagram illustrating an example of the information processing system 200. In FIG. 2, the information processing system 200 includes the information processing apparatus 100 and one or more measuring instruments 201. In the information processing system 200, the information processing apparatus 100 and the measuring instrument 201 are connected via a wired or wireless network 210. Examples of the network 210 include a local area network (LAN), a wide area network (WAN), and the Internet.

The information processing apparatus 100 is a computer that acquires measurement information regarding movement of each leg of the patient serving as a measurement target u from the one or more measuring instruments 201. Among a plurality of candidate groups obtained by combining candidates for the point of time at which each leg of the patient lands, the information processing apparatus 100 specifies a likely candidate group of a combination of the points of time at which each leg of the patient has actually landed. Examples of the information processing apparatus 100 include a server, a personal computer (PC), a notebook PC, a tablet terminal, a smartphone, and a wearable terminal.

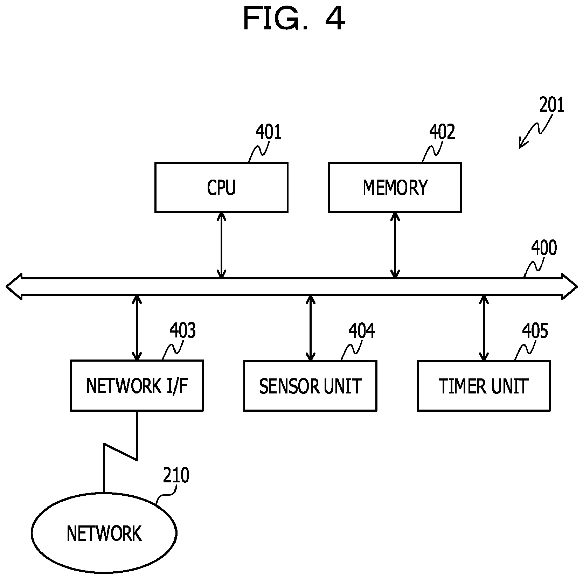

The measuring instrument 201 is a computer attached to the patient serving as the measurement target u. The measuring instrument 201 generates measurement information and transmits the measurement information to the information processing apparatus 100. The measuring instrument 201 includes, for example, a sensor unit illustrated in FIG. 4, generates, as measurement information, sensor data in which a measurement value of the sensor unit and a measurement time at which the measurement value of the sensor unit is obtained are associated with each other, and transmits the sensor data to the information processing apparatus 100. Specifically, the measuring instrument 201 generates, as the measurement information, sensor data in which angular velocity in a part of the patient to which the measuring instrument is attached is arranged in time series and sensor data in which acceleration in the part is arranged in time series and transmits the sensor data to the information processing apparatus 100.

In the following description, sensor data in which angular velocity is arranged in time series is also referred to as "gyro data". Sensor data in which acceleration is arranged in time series is also referred to as "acceleration data". The measuring instrument 201 is, for example, a sensor device. The sensor device is specifically a device referred to as "motion sensor". The measuring instrument 201 may be, for example, a smartphone or a wearable terminal.

Further, in a case where there are a plurality of measuring instruments 201, one of the measuring instruments 201 may be embedded in the floor. The measuring instrument 201 is embedded in the floor, generates measurement information regarding reaction force from the ground to the patient, and transmits the measurement information to the information processing apparatus 100. The measurement information regarding the reaction force from the ground to the patient is sensor data in which the reaction force from the ground to the patient is arranged in time series. In the following description, sensor data in which reaction force is arranged in time series is also referred to as "reaction force data".

In order to specify which patient's reaction force data the generated reaction force data is, the measuring instrument 201 may transmit the reaction force data to another measuring instrument 201 attached to the patient and cause the another measuring instrument 201 to transfer the reaction force data to the information processing apparatus 100. Further, the measuring instrument 201 may receive identification information for specifying a patient approaching the measuring instrument 201 and transmit the identification information in association with the reaction force data to the information processing apparatus 100. The measuring instrument 201 is, for example, a reaction force sensor device.

Herein, the case where the measuring instrument 201 creates sensor data such as gyro data, acceleration data, and reaction force data has been described. However, the present invention is not limited thereto. For example, the information processing apparatus 100 may sequentially receive, from the measuring instrument 201, correspondence information in which a measurement value of the sensor unit of the measuring instrument 201 and a measurement time at which the measurement value of the sensor unit is obtained are associated with each other, compile the correspondence information, and generate sensor data.

Herein, the case where the information processing apparatus 100 and the measuring instrument 201 are different devices has been described. However, the present invention is not limited thereto. For example, the information processing apparatus 100 may be integrated with the measuring instrument 201. Herein, the case where one measuring instrument 201 is attached to a living body has been described. However, the present invention is not limited thereto. For example, a plurality of measuring instruments 201 may be attached to a living body.

The information processing system 200 may further include a display device. In this case, the information processing apparatus displays the predetermined feature value regarding the patient on the display device. Examples of the display device include a PC, a notebook PC, a tablet terminal, and a smartphone.

Furthermore, the information processing system 200 may further include a relay device. In this case, the relay device receives measurement information from the one or more measuring instruments 201 and collectively transmits the received measurement information to the information processing apparatus. Examples of the relay device include a PC, a notebook PC, a tablet terminal, and a smartphone.

For example, the information processing system 200 is applied to a case of implementing a watching service for grasping a condition of the patient or is applied to a case of implementing a service for a personal health record (PHR).

(Exemplary Hardware Configuration of Information Processing Apparatus 100)

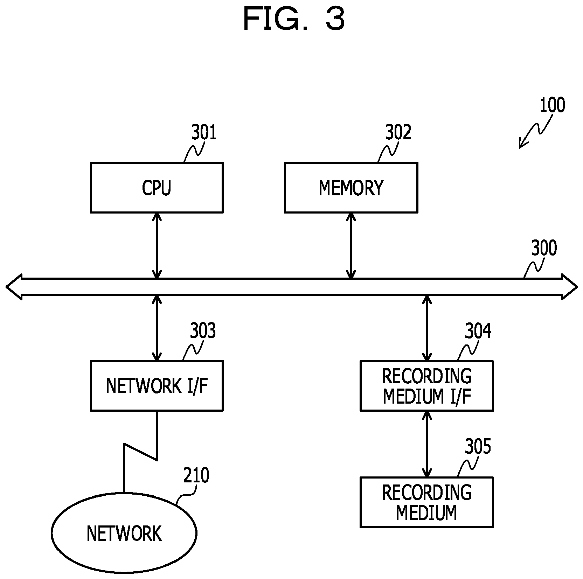

Next, an exemplary hardware configuration of the information processing apparatus 100 included in the information processing system 200 illustrated in FIG. 2 will be described with reference to FIG. 3.

FIG. 3 is a block diagram illustrating an exemplary hardware configuration of the information processing apparatus 100. In FIG. 3, the information processing apparatus 100 includes a central processing unit (CPU) 301, a memory 302, a network interface (I/F) 303, a recording medium I/F 304, and a recording medium 305. Furthermore, each of those components is interconnected by a bus 300.

Herein, the CPU 301 performs overall control of the information processing apparatus 100. The memory 302 includes, for example, a read only memory (ROM), a random access memory (RAM), and a flash ROM. Specifically, for example, the flash ROM or the ROM stores various programs, while the RAM is used as a work area for the CPU 301. A program stored in the memory 302 is loaded into the CPU 301 to cause the CPU 301 to execute coded processing.

The network I/F 303 is connected to the network 210 through a communication line, and is connected to another computer through the network 210. In addition, the network I/F 303 manages an interface between the network 210 and an inside, and controls input-output of data from another computer. Examples of the network I/F 303 include a modem and a LAN adapter.

The recording medium I/F 304 controls read and write of data toward the recording medium 305 under the control of the CPU 301. Examples of the recording medium I/F 304 include a disk drive, a solid state drive (SSD), and a universal serial bus (USB) port. The recording medium 305 is a nonvolatile memory that stores data written under the control of the recording medium I/F 304. Examples of the recording medium 305 include a disk, a semiconductor memory, and a USB memory. The recording medium 305 may be removably installed on the information processing apparatus 100.

The information processing apparatus 100 may further include a keyboard, a mouse, a display, a printer, a speaker, or a touch panel, for example, in addition to the components described above. In addition, the information processing apparatus 100 may omit the recording medium I/F 304 or the recording medium 305.

(Exemplary Hardware Configuration of Measuring Instrument 201)

Next, an exemplary hardware configuration of the measuring instrument 201 included in the information processing system 200 illustrated in FIG. 2 will be described with reference to FIG. 4.

FIG. 4 is a block diagram illustrating an exemplary hardware configuration of the measuring instrument 201. In FIG. 4, the measuring instrument 201 includes a CPU 401, a memory 402, a network I/F 403, a sensor unit 404, and a timer unit 405. In addition, each of the components is interconnected by a bus 400.

Herein, the CPU 401 performs overall control of the measuring instrument 201. The memory 402 includes, for example, a ROM, a RAM, and a flash ROM. Specifically, for example, the flash ROM or the ROM stores various programs, while the RAM is used as a work area for the CPU 401. A program stored in the memory 402 is loaded into the CPU 401 to cause the CPU 401 to execute coded processing.

The network I/F 403 is connected to the network 210 through a communication line, and is connected to another computer through the network 210. In addition, the network I/F 403 manages an interface between the network 210 and an inside, and controls input-output of data from another computer. Examples of the network I/F 403 include a communication circuit corresponding to Wi-Fi (registered trademark) and a communication circuit corresponding to Bluetooth (registered trademark). The network I/F 403 may be, for example, a communication circuit corresponding to the 3rd Generation (3G).

The sensor unit 404 measures a condition of the measuring instrument 201. The sensor unit 404 measures, for example, at least one of a position, movement, and an orientation of the measuring instrument 201. Specifically, the sensor unit 404 includes at least one of an acceleration sensor, an angular velocity sensor, a terrestrial magnetism sensor, an optical sensor, a vibration sensor, and the like. In addition, the sensor unit 404 may include a global positioning systems (GPS) receiver, and may detect GPS coordinates of the measuring instrument 201. The timer unit 405 measures a current time.

The measuring instrument 201 may further include a keyboard, a mouse, a display, a printer, a speaker, or a touch panel, for example, in addition to the components described above. In addition, the measuring instrument 201 may further include a recording medium I/F or a recording medium, in addition to the components described above. The recording medium may be removably installed on the measuring instrument 201.

(Exemplary Functional Configuration of Information Processing Apparatus 100)

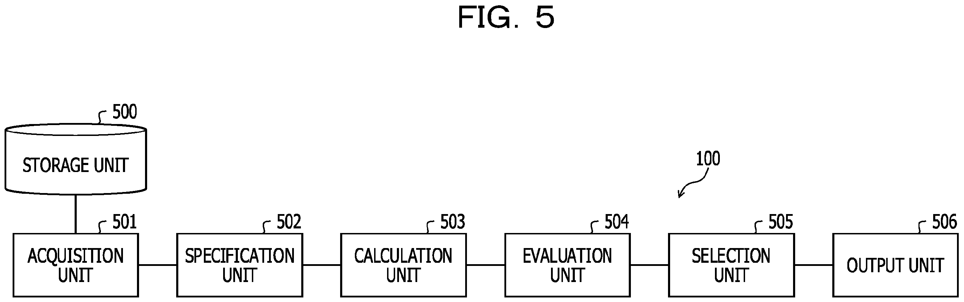

Next, an exemplary functional configuration of the information processing apparatus 100 will be described with reference to FIG. 5.

FIG. 5 is a block diagram illustrating an exemplary functional configuration of the information processing apparatus 100. The information processing apparatus 100 includes a storage unit 500, an acquisition unit 501, a specification unit 502, a calculation unit 503, an evaluation unit 504, a selection unit 505, and an output unit 506.

For example, the storage unit 500 is implemented by a storage area such as the memory 302 or the recording medium 305 illustrated in FIG. 3. The acquisition unit 501 to the output unit 506 have functions as a control unit. Specifically, for example, the acquisition unit 501 to the output unit 506 implement functions thereof by causing the CPU 301 to execute a program stored in the storage area such as the memory 302 or the recording medium 305 illustrated in FIG. 3, or by the network I/F 303. A processing result of each functional unit is stored in, for example, the storage area such as the memory 302 or the recording medium 305 illustrated in FIG. 3.

The storage unit 500 stores measurement information regarding the movement of each leg of the subject. The subject is, for example, a patient. The storage unit 500 stores, for example, sensor data in which a measurement value of the sensor unit of the measuring instrument 201 and a measurement time at which the measurement value is obtained are associated with each other and arranged in time series.

The measurement value includes, for example, at least one of the angular velocity on a sagittal plane, the angular velocity on a traverse plane, and the angular velocity on a coronal plane. The measurement value may include, for example, at least one of acceleration in a vertical direction, acceleration in a lateral direction, and acceleration in a longitudinal direction. The measurement value may include, for example, magnitude and a position of vibration. The storage unit 500 may further store measurement information regarding movement of a waist of the subject. With this, the storage unit 500 can refer to sensor data.

The storage unit 500 stores an evaluation method for evaluating a degree of appearance of a predetermined relationship between landing positions of both the legs of the patient. The predetermined relationship is, for example, a relationship in which the landing position of the leg serving as the swing leg of the patient falls within a predetermined range in front of the landing position of the leg serving as the support leg of the patient. The predetermined relationship may be, for example, a relationship in which the landing position of the leg serving as the swing leg of the patient falls within a predetermined range in front of a start point existing at a predetermined distance behind the landing position of the leg serving as the support leg of the patient. The storage unit 500 stores, for example, Expressions (1) to (15) described below as expressions for use in the evaluation method.

The acquisition unit 501 acquires measurement information regarding the movement of each leg of the patient. For example, the acquisition unit 501 receives sensor data of the angular velocity and acceleration of the shin of the left leg of the patient from the measuring instrument 201 attached to the shin of the left leg of the patient and receives sensor data of the angular velocity and acceleration of the shin of the right leg of the patient from the measuring instrument 201 attached to the shin of the right leg of the patient. With this, the acquisition unit 501 can use the sensor data to specify the step length of the patient.

The acquisition unit 501 may further acquire measurement information regarding the movement of the waist of the patient. For example, the acquisition unit 501 receives sensor data of acceleration of the waist of the patient from the measuring instrument 201 attached to the waist of the patient. The acquisition unit 501 may further acquire measurement information regarding reaction force from the ground to the patient. The acquisition unit 501 may further acquire a travel distance of the patient. With this, the acquisition unit 501 can refer to information used to further improve accuracy of specifying the step length of the patient.

Based on the acquired measurement information, the specification unit 502 specifies, for each step in the walking period of the patient, one or more candidates for the point of time corresponding to landing of the leg serving as the swing leg of the patient. For example, based on the sensor data, the specification unit 502 specifies, as a candidate, a point of time at which the acceleration in the vertical direction is equal to or larger than a threshold in a downward direction within a predetermined time period from a point of time of movement at which the angular velocity exceeds a threshold and reaches the maximum value. With this, the specification unit 502 can specify a candidate for the point of time at which each leg lands.

The specification unit 502 specifies a plurality of candidate groups obtained by selecting one of the one or more candidates specified for each step in the walking period and combining the selected candidates. With this, the specification unit 502 can specify a candidate group that may be a combination of the points of time at which each leg of the patient lands.

The specification unit 502 may set, as the walking period, a period in which walking movement is performed by the patient and the number of steps of the patient is less than a predetermined number of steps. With this, the number of steps of the patient is less than the predetermined number of steps and accumulation of errors tends to be reduced, and thus the specification unit 502 can restrain calculation accuracy of the calculation unit 503 from being lowered.

The calculation unit 503 calculates the step length of each step in the walking period for each of the plurality of specified candidate groups, and, based on the calculated step length of each step in the walking period, calculates the cumulative travel distance of the leg serving as the swing leg of the patient for each step in the walking period.

For example, in the first step or the second step, the calculation unit 503 sets the step length of the leg in the first step or the second step as the cumulative travel distance of the leg. For example, in the third step or the following steps, the calculation unit 503 adds the step length of the leg serving as the swing leg in the step to the cumulative travel distance of the leg. With this, the calculation unit 503 can acquire a reference of evaluation of the evaluation unit 504.

Based on the cumulative travel distance calculated for each step in the walking period, the evaluation unit 504 evaluates, for each candidate group, a degree of appearance of a predetermined relationship in the walking period. The predetermined relationship is a relationship in which the landing position of the leg serving as the swing leg of the patient falls within a predetermined range of the landing position of the leg serving as the support leg of the patient.

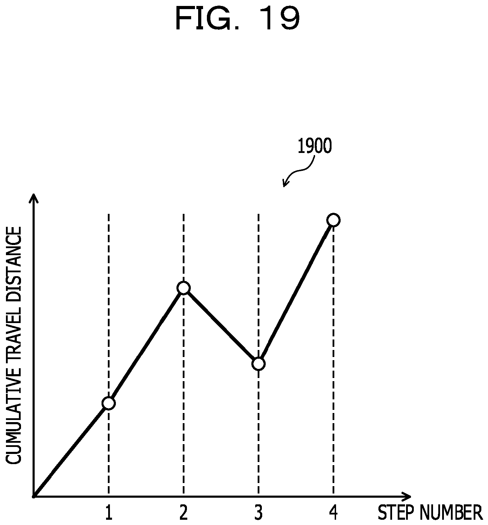

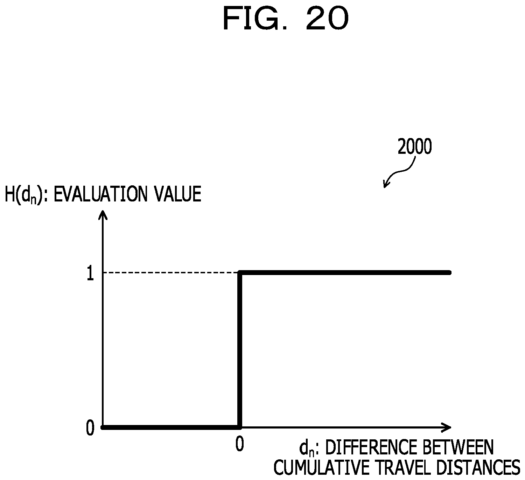

For example, the evaluation unit 504 uses, as the predetermined range, a range from a start point that is the landing position of the leg serving as the support leg of the patient to an end point that is a position existing at the first distance in front of the start point. Then, for each candidate group, the evaluation unit 504 determines whether or not the predetermined relationship has appeared for each step in the walking period and calculates an evaluation value of the candidate group so that the evaluation value of the candidate group is increased as the number of times of appearance of the predetermined relationship is increased. Specifically, the evaluation unit 504 calculates the evaluation value as described below with reference to FIGS. 19 and 20. With this, the evaluation unit 504 can evaluate whether or not each candidate group is likely to be a combination of the points of time at which each leg of the patient lands.

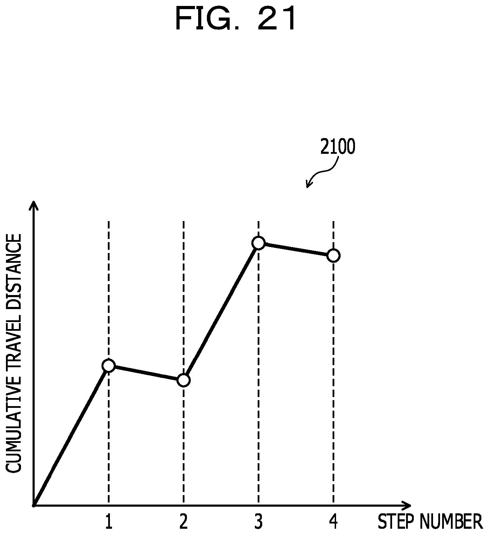

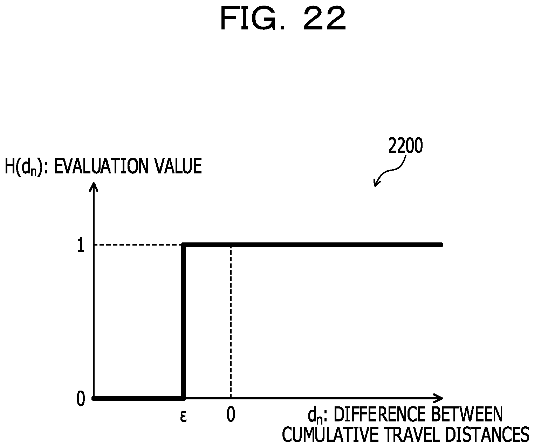

For example, the evaluation unit 504 may use, as the predetermined range, a range from a start point that is a position existing at a second distance behind the landing position of the leg serving as the support leg of the patient to an end point that is a position existing at the first distance in front of the start point. Then, for each candidate group, the evaluation unit 504 determines whether or not the predetermined relationship has appeared for each step in the walking period and calculates an evaluation value of the candidate group so that the evaluation value of the candidate group is increased as the number of times of appearance of the predetermined relationship is increased. Specifically, the evaluation unit 504 calculates the evaluation value as described below with reference to FIGS. 21 and 22. With this, the evaluation unit 504 can evaluate whether or not each candidate group is likely to be a combination of the points of time at which each leg of the patient lands.

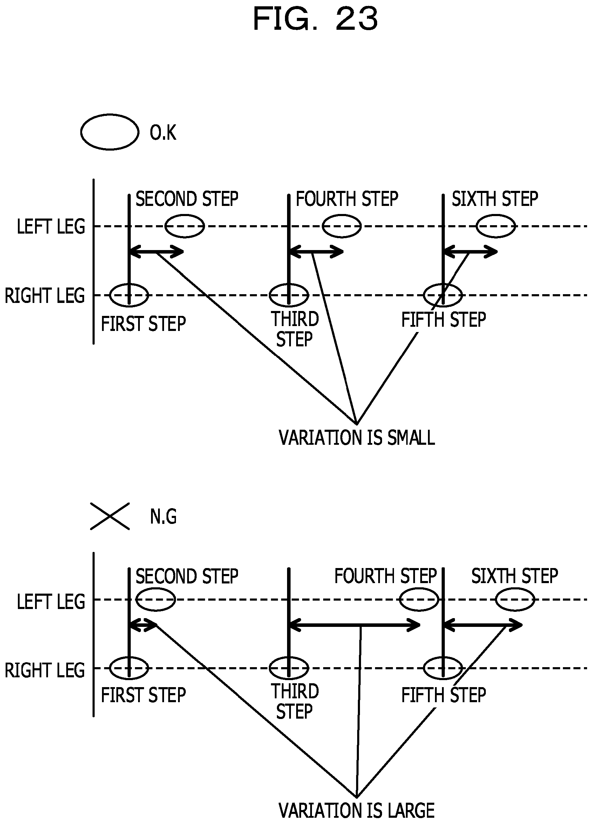

Further, for each candidate group, the evaluation unit 504 may calculate the evaluation value of the candidate group so that the evaluation value of the candidate group is increased as a difference between the cumulative travel distances of both the legs of the patient is constant in the walking period. Specifically, the evaluation unit 504 calculates the evaluation value as described below with reference to FIG. 23. With this, the evaluation unit 504 can improve accuracy of selecting a candidate group.

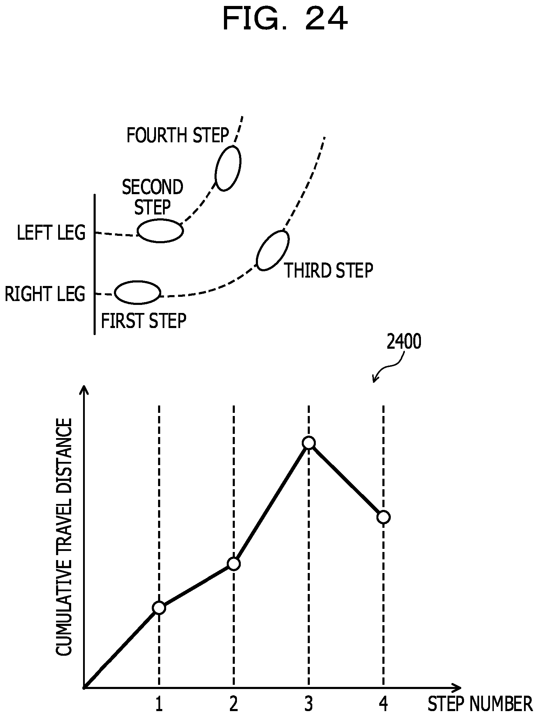

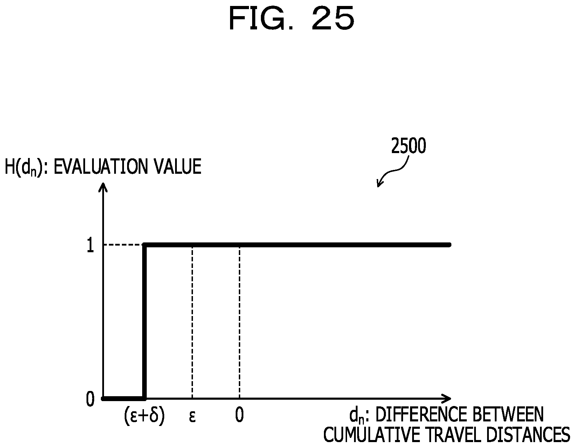

For example, for each candidate group, the evaluation unit 504 may set, for each step in the walking period, a second distance according to a rotation direction and a degree of rotation of the leg serving as the swing leg of the patient and may determine whether or not the predetermined relationship has appeared. Specifically, the evaluation unit 504 calculates the evaluation value as described below with reference to FIGS. 24 and 25. With this, the evaluation unit 504 can evaluate whether or not each candidate group is likely to be a combination of the points of time at which each leg of the patient lands.

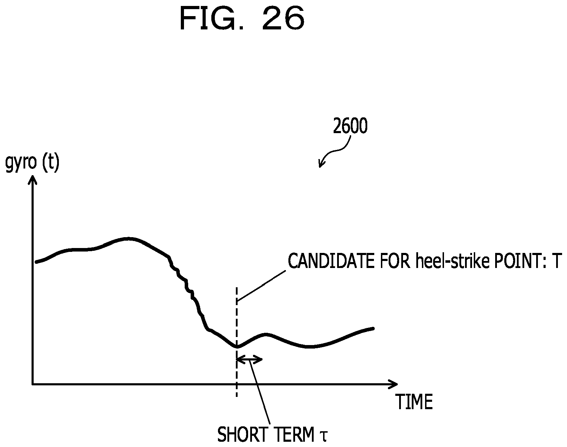

The evaluation unit 504 may further calculate, for each candidate group, a predetermined feature value for each candidate on the basis of measurement information regarding the movement of the waist of the patient or measurement information regarding the movement of each leg of the patient and calculate the evaluation value of the candidate group on the basis of the calculated predetermined feature value of each candidate. Specifically, the evaluation unit 504 calculates the evaluation value as described below with reference to FIGS. 26 to 32. With this, the evaluation unit 504 can improve accuracy of selecting a candidate group.

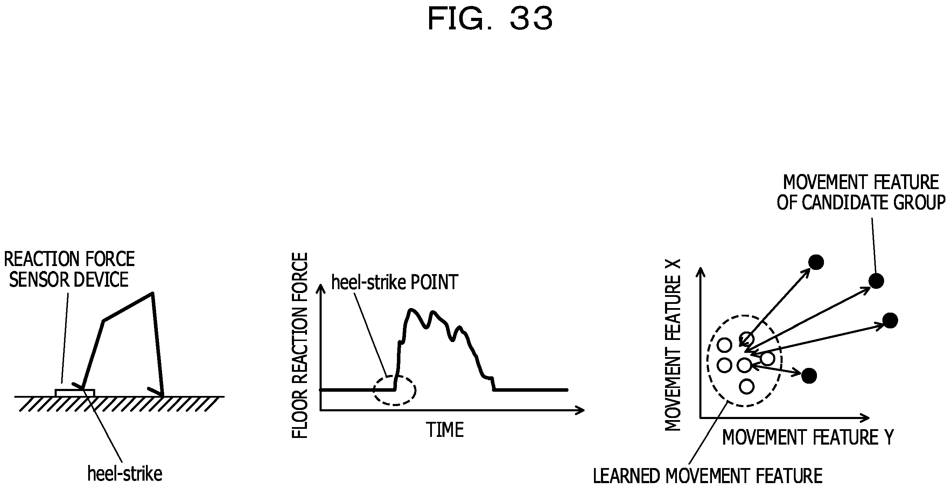

The evaluation unit 504 may further calculate, for each candidate group, a predetermined feature value for each candidate on the basis of measurement information regarding reaction force or measurement information regarding the movement of each leg of the patient and calculate the evaluation value of the candidate group on the basis of the calculated predetermined feature value of each candidate. Specifically, the evaluation unit 504 calculates the evaluation value as described below with reference to FIG. 33. With this, the evaluation unit 504 can improve accuracy of selecting a candidate group.

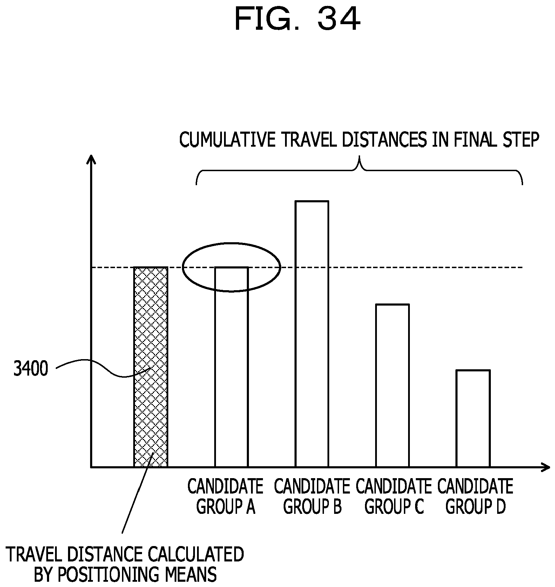

The evaluation unit 504 may further calculate, for each candidate group, the evaluation value of the candidate group on the basis of a difference between the cumulative travel distance of the leg serving as the swing leg of the patient in the final step in the walking period and the acquired travel distance of the patient. Specifically, the evaluation unit 504 calculates the evaluation value as described below with reference to FIG. 34. With this, the evaluation unit 504 can improve accuracy of selecting a candidate group.

The selection unit 505 selects one of the plurality of candidate groups on the basis of the evaluation result. For example, the selection unit 505 selects one of the plurality of candidate groups on the basis of the calculated evaluation value of each candidate group. Specifically, the selection unit 505 specifies the maximum value of the sum of the calculated evaluation values of each candidate group and selects a candidate group having the maximum value.

The output unit 506 outputs information on the candidate group selected by the selection unit 505. The output is implemented as, for example, display on a display, print output to a printer, transmission to an external device by the network I/F 303, or storage to the storage area such as the memory 302 and the recording medium 305.

(One Example of Flow in which Information Processing Apparatus 100 Selects Candidate Group)

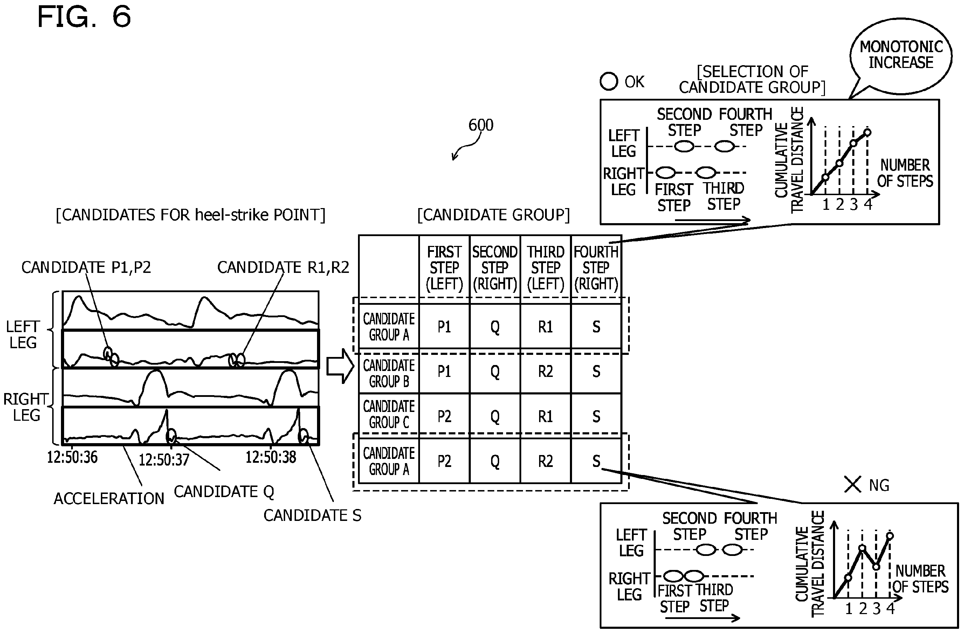

Next, an example of a flow in which the information processing apparatus 100 selects a candidate group will be described with reference to FIG. 6.

FIG. 6 is an explanatory diagram illustrating an example of a flow in which the information processing apparatus 100 selects a candidate group. In FIG. 6, the information processing apparatus 100 acquires sensor data of the angular velocity and acceleration of the left shin and sensor data of the angular velocity and acceleration of the right shin.

Based on the sensor data, the information processing apparatus 100 specifies candidates P1, P2, R1, and R2 of the point of time at which the left leg performs heel-strike in the walking period and specifies candidates Q and S of the point of time at which the right leg performs heel-strike in the walking period. In the following description, the point of time at which each leg performs heel-strike is also referred to as "heel-strike point".

The information processing apparatus 100 specifies candidate groups A to D by selecting one of the candidates corresponding to each step in the walking period and combining the selected candidates. The information processing apparatus 100 stores the specified candidate groups A to D by using a candidate group management table 600. The information processing apparatus 100 calculates the step length of each step in the walking period on the basis of each of the candidate groups A to D. Based on the calculated step length, the information processing apparatus 100 calculates the cumulative travel distance in each of the number of steps.

The information processing apparatus 100 selects a candidate group that satisfies the relationship in which the landing position of the leg serving as the swing leg falls within a predetermined range in front of the landing position of the leg serving as the support leg. Herein, in a case where the relationship in which the landing position of the leg serving as the swing leg falls within the predetermined range in front of the landing position of the leg serving as the support leg is satisfied, the cumulative travel distance is monotonically increased in each step.

Therefore, for example, the information processing apparatus 100 selects a candidate group in which the cumulative travel distance is monotonically increased in each step as a candidate group that satisfies the relationship in which the landing position of the leg serving as the swing leg falls within the predetermined range in front of the landing position of the leg serving as the support leg. The information processing apparatus 100 calculates the step length of the patient on the basis of the selected candidate group. With this, the information processing apparatus 100 can improve accuracy of calculating the step length of the patient.

Further, two or more candidate groups satisfy the above-mentioned relationship in some cases, and thus it is difficult for the information processing apparatus 100 to select one candidate group. In this case, the information processing apparatus 100 may further improve the accuracy of selecting the candidate group also on the basis of information other than the cumulative travel distance. The information processing apparatus 100 improves the accuracy of selecting a candidate group by using, for example, a difference between the cumulative travel distances of both the legs of the patient, as described below with reference to FIG. 23.

Further, for example, the information processing apparatus 100 may improve the accuracy of selecting a candidate group by using a predetermined feature value of each candidate, as described below with reference to FIGS. 26 to 32. For example, the information processing apparatus 100 may improve the accuracy of selecting a candidate group by using measurement information regarding the movement of the waist of the patient, as described below with reference to FIGS. 26 to 32.

Further, for example, the information processing apparatus 100 may improve the accuracy of selecting a candidate group by using measurement information regarding the reaction force from the ground to the patient, as described below with reference to FIG. 33. Furthermore, for example, the information processing apparatus 100 may improve the accuracy of selecting a candidate group by using the travel distance of the patient as described below with reference to FIG. 34.

(Specific Examples in which Information Processing Apparatus 100 Selects Candidate Group)

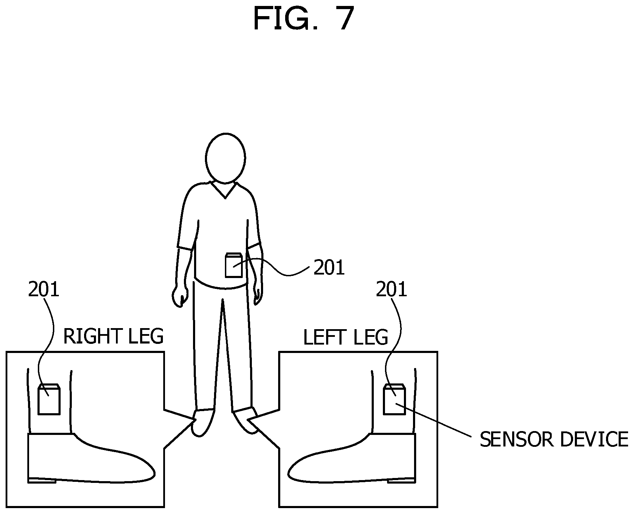

Next, specific examples in which the information processing apparatus 100 selects a candidate group will be described with reference to FIGS. 7 to 36.

FIGS. 7 to 36 are explanatory diagrams illustrating specific examples in which the information processing apparatus 100 selects a candidate group. In FIG. 7, the patient wears the measuring instruments 201 on his/her right leg, left leg, and waist. For example, the patient wears the measuring instruments 201 on the shin of the right leg and the shin of the left leg. Each measuring instrument 201 measures angular velocity and acceleration in a part of the patient to which the measuring instrument is attached and generates gyro data and acceleration data as sensor data in which a measurement value and a measurement time are associated with each other.

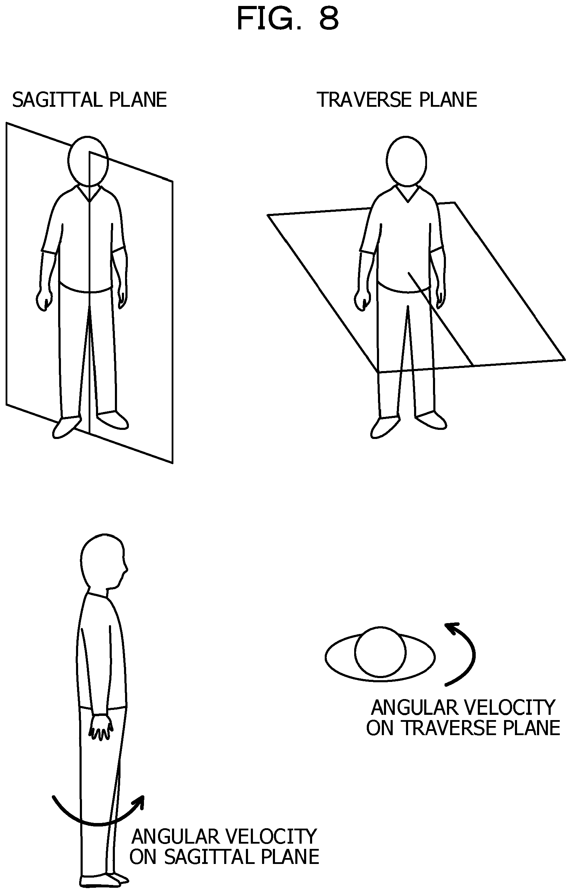

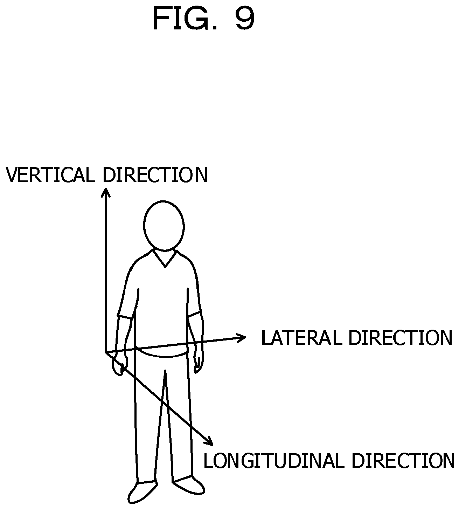

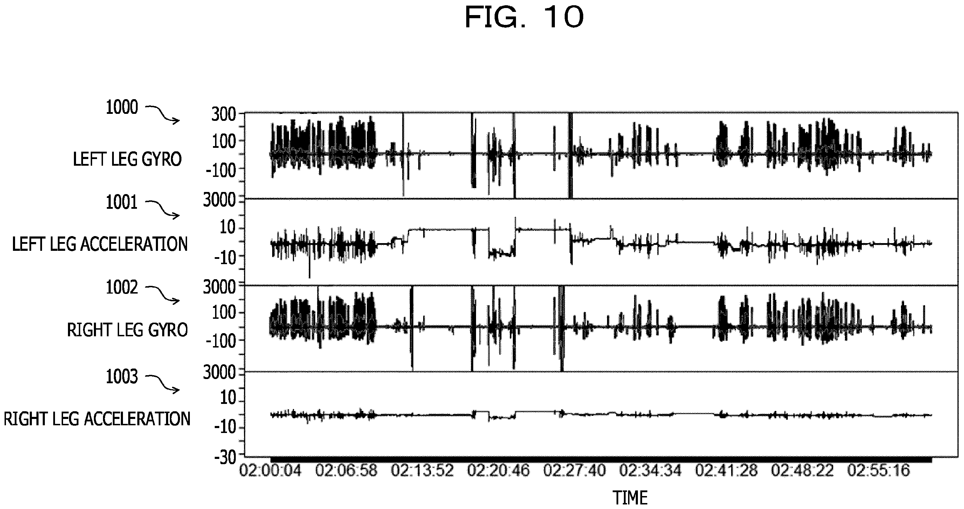

The information processing apparatus 100 receives the gyro data, the acceleration data, and the like generated by the measuring instrument 201. Specific examples of the gyro data and acceleration data will be described below with reference to FIG. 10. In a case where the measuring instrument 201 serving as a reaction force sensor device is attached, the information processing apparatus 100 may receive reaction force data from the measuring instrument 201. Next, description of FIGS. 8 and 9 will be made, and the contents of measurement by the measuring instruments 201 will be described.

As illustrated in FIG. 8, in a case where the angular velocity is measured, the measuring instruments 201 measure, for example, the angular velocity on the sagittal plane, the angular velocity on the traverse plane, and the angular velocity on the coronal plane. With this, for example, the measuring instrument 201 generates, as sensor data, gyro data in which the angular velocity on the sagittal plane is arranged in time series, gyro data in which the angular velocity on the traverse plane is arranged in time series, and the like. A positive direction of each angular velocity corresponds to a direction of the arrow in FIG. 8.

As illustrated in FIG. 9, in a case where the acceleration is measured, the measuring instruments 201 measure, for example, the acceleration in the vertical direction, the acceleration in the lateral direction, and the acceleration in the longitudinal direction. In the following description, the acceleration in the vertical direction, the acceleration in the lateral direction, and the acceleration in the longitudinal direction are also referred to as "vertical acceleration", "lateral acceleration", and "longitudinal acceleration", respectively. With this, for example, the measuring instruments 201 generate, as sensor data, acceleration data in which the vertical acceleration is arranged in time series, acceleration data in which the lateral acceleration is arranged in time series, and acceleration data in which the longitudinal acceleration is arranged in time series. Next, description of FIG. 10 will be made.

As illustrated in FIG. 10, examples of sensor data received by the information processing apparatus 100 include left leg gyro data 1000, left leg acceleration data 1001, right leg gyro data 1002, right leg acceleration data 1003, and the like.

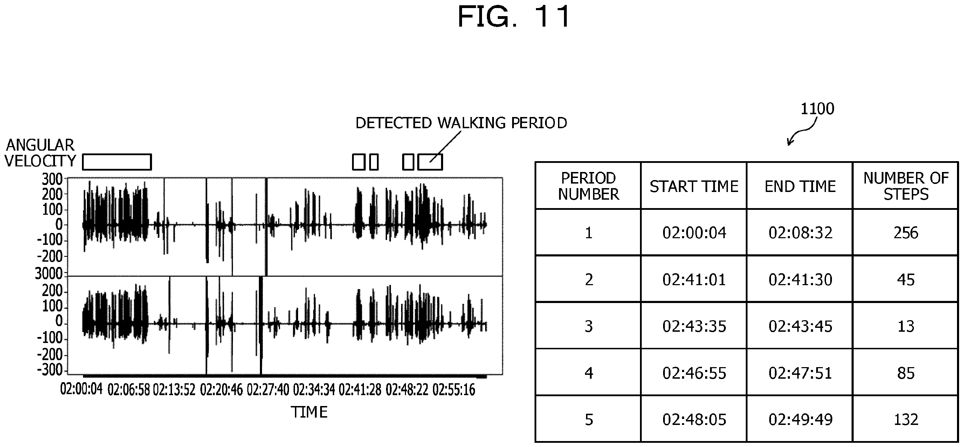

The gyro data 1000 shows a waveform in which the angular velocity on the sagittal plane and the angular velocity on the traverse plane regarding the left leg are collectively arranged in time series. The acceleration data 1001 shows a waveform in which the vertical acceleration of the left leg is arranged in time series. The gyro data 1002 shows a waveform in which the angular velocity on the sagittal plane and the angular velocity on the traverse plane regarding the right leg are collectively arranged in time series. The acceleration data 1003 shows a waveform in which the vertical acceleration of the right leg is arranged in time series. Next, description of FIG. 11 will be made.

In FIG. 11, the information processing apparatus 100 specifies the walking period on the basis of the gyro data of the sagittal plane of the left leg and the gyro data of the sagittal plane of the right leg. For example, the information processing apparatus 100 applies a low-pass filter to each gyro data, detects a point of time at which the angular velocity exceeds a threshold and reaches the maximum value, and specifies, as a candidate period of the walking period, a period in which average energy at the detected point of time is large. In the following description, a point of time at which the angular velocity exceeds a threshold and reaches the maximum value is also referred to as "peak point".

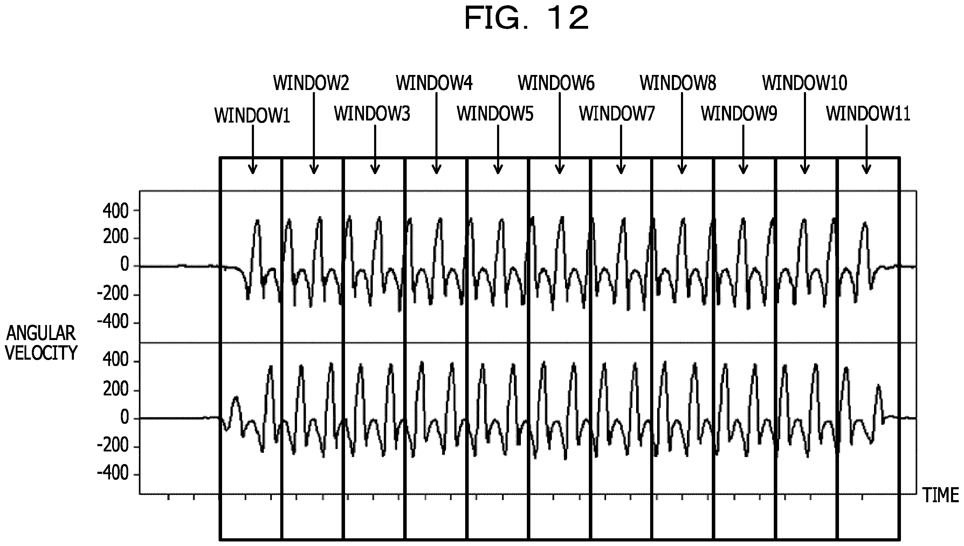

In a case where feature values around the peak point satisfy a predetermined condition in the candidate period, the information processing apparatus 100 specifies the candidate period as the walking period. When the information processing apparatus 100 specifies the walking period, the information processing apparatus 100 stores a start time and an end time of the walking period and the number of steps in the walking period by using a walking period management table 1100. Next, description of FIG. 12 will be made.

In FIG. 12, the information processing apparatus 100 divides the specified walking period into a plurality of partial periods. For example, the information processing apparatus 100 shifts a window having a window width W by a shift width W and extracts a partial period corresponding to the window. The information processing apparatus 100 selects a candidate group for each partial period and calculates the step length. However, in the following description, only one of the partial periods will be described.

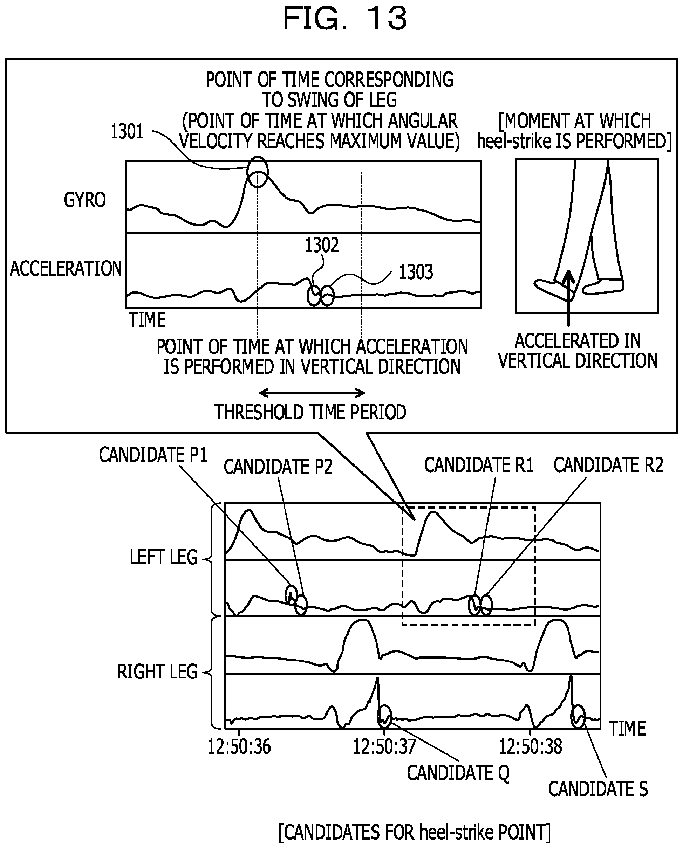

With this, the information processing apparatus 100 can restrain an increase in the number of candidates included in a candidate group to be specified in the subsequent processing and can also restrain an increase in throughput, as compared with a case where the walking period is employed as a unit of processing. In the example of FIG. 12, in a case where W = 4 steps is satisfied, the information processing apparatus 100 extracts eleven partial periods as a result of division of the walking period. Next, description of FIG. 13 will be made.

In FIG. 13, the information processing apparatus 100 specifies a candidate for the heel-strike point for each step in one of the partial periods. Herein, in one step, the leg performs heel-strike after the leg swings, and thus a characteristic of the heel-strike tends to appear after the angular velocity reaches the maximum value because of the swing. Further, reaction force from the ground acts at a moment of the heel-strike, and the measuring instrument 201 has a property of measuring the vertical acceleration for a moment. Therefore, the characteristic of the heel-strike is, for example, such that acceleration equal to or larger than a threshold is generated in the vertical direction.

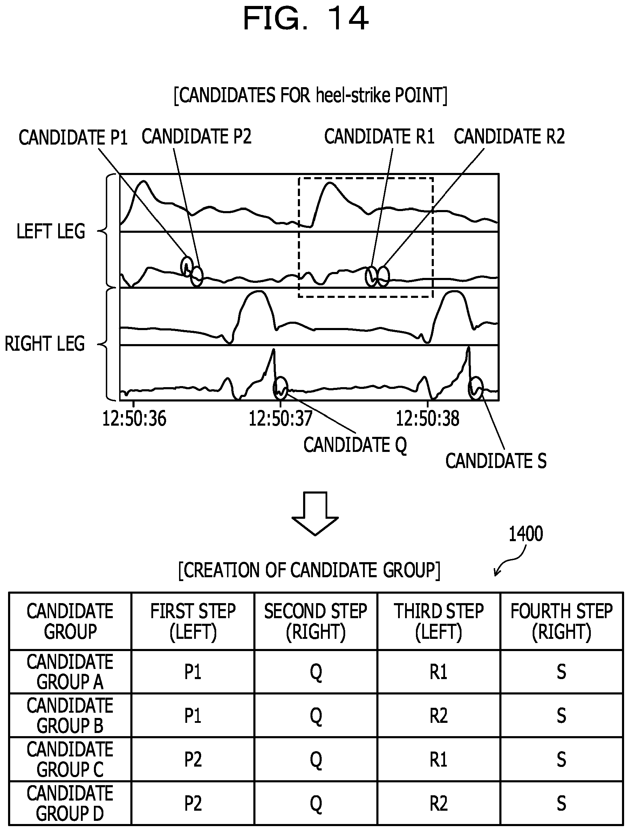

Therefore, candidates for the heel-strike point are points of time 1302, 1303, and the like at which acceleration equal to or larger than the threshold is generated in the vertical direction within a predetermined time after a point of time 1301 at which the angular velocity of the leg reaches the maximum value. Specifically, the information processing apparatus 100 specifies, in the walking period, the candidates P1, P2, R1, and R2 of the heel-strike point of the left leg and the candidates Q and S of the heel-strike point of the right leg. Next, description of FIG. 14 will be made.

In FIG. 14, the information processing apparatus 100 specifies a plurality of candidate groups on the basis of the candidates P1, P2, R1, and R2 of the heel-strike point of the left leg and the candidates Q and S of the heel-strike point of the right leg. Herein, there are two candidates P1 and P2 in the first step, two candidates R1 and R2 in the third step, and one candidate in the second and fourth steps, and therefore $2 \times 1 \times 2 \times 1 = 4$ candidate groups are specified.

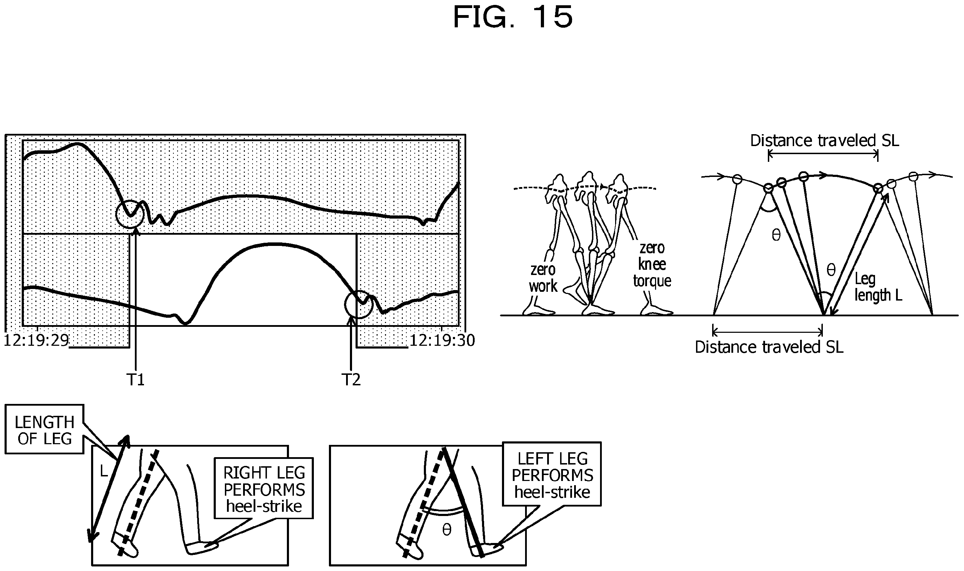

For example, the information processing apparatus 100 specifies the candidate groups A to D by selecting one of the candidates corresponding to each step in the walking period and combining the selected candidates. The information processing apparatus 100 stores the specified candidate groups A to D by using a candidate group management table 1400. Next, description of FIG. 15 will be made.

In FIG. 15, the information processing apparatus 100 calculates the step length of each step in the walking period on the basis of each of the candidate groups A to D. For example, the information processing apparatus 100 can obtain a step length SL of the leg serving as the swing leg in a step by multiplying, by a coefficient, an integral value of the angular velocity on the sagittal plane from a heel-strike point T1 of the leg serving as the support leg to a heel-strike point T2 of the leg serving as the swing leg.

Specifically, in a case where each leg is regarded as a bar, the integral value of the angular velocity on the sagittal plane from the heel-strike point T1 of the leg serving as the support leg to the heel-strike point T2 of the leg serving as the swing leg corresponds to an angle indicating a degree of opening of a hip joint. Therefore, considering a triangle in which a line of each leg and a line of the step length SL are combined, there is such a property that the step length SL can be calculated by using a trigonometric function. The information processing apparatus 100 calculates the step length SL by using, for example, the following Expression (1).

[Expression 1]

$$SL \cong 2L \times \sin(\theta/2) = 2L \times \sin\left(\sum_{t=T_1}^{T_2} gyro(t)/2\right) \quad \ldots (1)$$

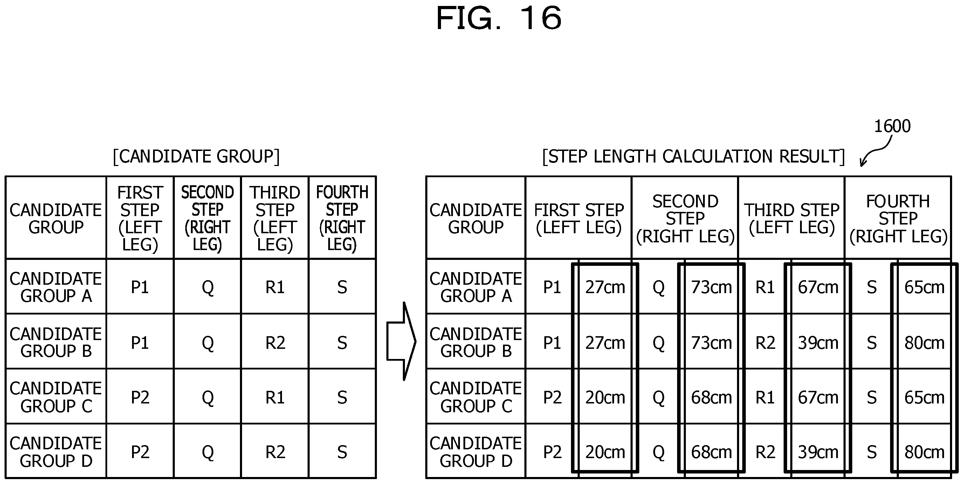

Herein, the symbol "SL" represents a step length. The symbol "L" represents length of the leg. The term "gyro(t)" represents angular velocity at a time t. The symbol "T1" represents a heel-strike point of the leg serving as the support leg. The symbol "T2" represents a heel-strike point of the leg serving as the swing leg. Next, description of FIG. 16 will be made.

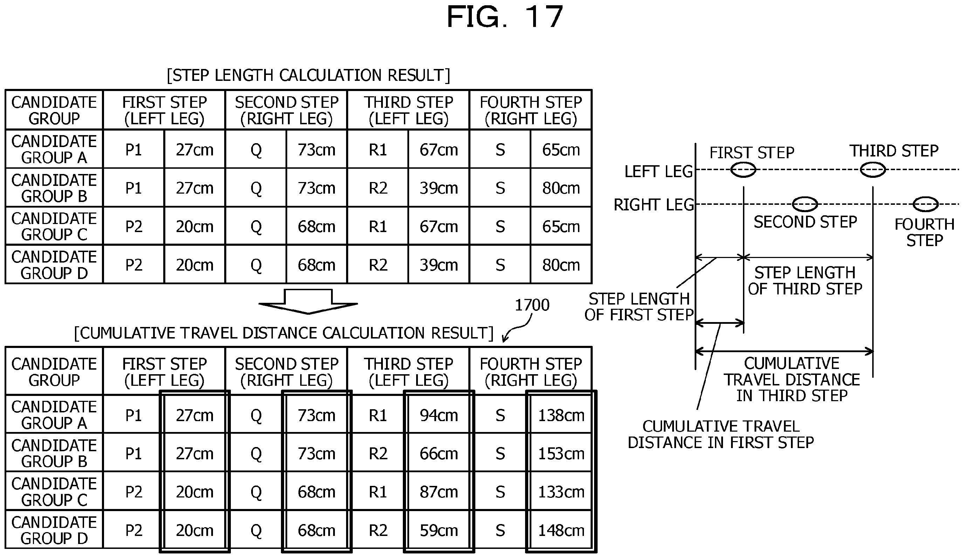

In FIG. 16, the information processing apparatus 100 refers to the candidate group management table 1400, specifies the heel-strike point, and calculates the step length of each step in the walking period on the basis of each of the candidate groups A to D. The information processing apparatus 100 stores the step length of each step calculated for each of the candidate groups A to D in association with each of the candidate groups A to D by using a step length management table 1600. Next, description of FIG. 17 will be made.

In FIG. 17, the information processing apparatus 100 calculates the cumulative travel distance in each of the number of steps on the basis of the calculated step length. For example, the information processing apparatus 100 calculates the cumulative travel distance of the left leg or the right leg serving as the swing leg in the Kth step.

For example, the left leg is the swing leg in the first step, and thus the information processing apparatus 100 sets the step length of the left leg in the first step as the cumulative travel distance of the left leg. For example, the right leg is the swing leg in the second step, and thus the information processing apparatus 100 sets the step length of the right leg in the second step as the cumulative travel distance of the right leg. For example, the left leg is the swing leg in the third step, and thus the information processing apparatus 100 adds the step length of the left leg in the third step to the cumulative travel distance of the left leg. For example, the right leg is the swing leg in the fourth step, and thus the information processing apparatus 100 adds the step length of the right leg in the fourth step to the cumulative travel distance of the right leg.

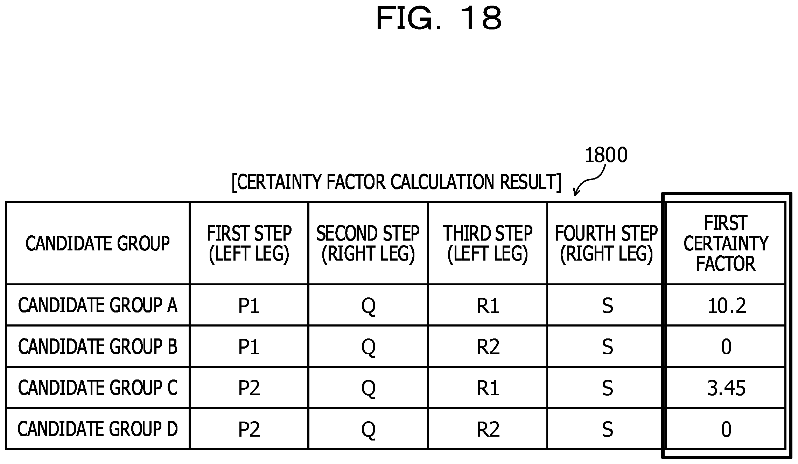

The information processing apparatus 100 stores the cumulative travel distance in each of the number of steps calculated for each of the candidate groups A to D in association with each of the candidate groups A to D by using a cumulative travel distance management table 1700. Next, description of FIG. 18 will be made.

In FIG. 18, the information processing apparatus 100 refers to the cumulative travel distance management table 1700 and calculates, for each of the candidate groups A to D, a first certainty factor indicating likelihood of a combination of the heel-strike points of both the legs of the patient. The information processing apparatus 100 stores the first certainty factor calculated for each of the candidate groups A to D in association with each of the candidate groups A to D by using a first certainty factor management table 1800. Next, description of FIGS. 19 and 20 will be made, and a specific example of calculating the first certainty factor will be described.

A graph 1900 in FIG. 19 associates the number of steps in a certain candidate group with a cumulative travel distance of the number of steps. A horizontal axis of the graph indicates the number of steps, and a vertical axis thereof indicates the cumulative travel distance. As illustrated in FIG. 19, the cumulative travel distance in the third step is smaller than the cumulative travel distance in the second step in the certain candidate group, and the cumulative travel distance is not monotonically increased.

Herein, the patient tends to walk while moving the swing leg ahead of the support leg, and thus the walking movement of the patient has such a property that a landing position of the leg serving as the swing leg falls within a predetermined range in front of a landing position of the leg serving as the support leg. In other words, in the walking movement of the patient, the cumulative travel distance in each step tends to be monotonically increased. In consideration of this tendency, the candidate group illustrated as the graph in FIG. 19 tends not to be a candidate group obtained by combining actual heel-strike points.

Therefore, the information processing apparatus 100 preferably calculates the first certainty factor of this candidate group so that the first certainty factor is low. For example, the information processing apparatus 100 calculates the first certainty factor so that the first certainty factor is higher as the relationship in which the landing position of the leg serving as the swing leg of the patient falls within the predetermined range in front of the landing position of the leg serving as the support leg of the patient appears and the cumulative travel distance is monotonically increased. Specifically, the information processing apparatus 100 calculates the first certainty factor by using the following Expressions (2) and (3).

[Expression 2]

$$H(d_n) = \begin{cases} 1(d_n \geq 0) \\ 0(d_n < 0) \end{cases} \quad \cdots (2)$$

The symbol "$d_n$" represents a difference obtained by subtracting the cumulative travel distance in the nth step from the cumulative travel distance in the $(n+1)$th step. The symbol "$H(d_n)$" represents a value indicating whether or not the heel-strike point in the nth step is likely to be an actual heel-strike point. In a case where $H(d_n)$ is 1, the heel-strike point is likely to be an actual heel-strike point. Herein, description of FIG. 20 will be made, and the above Expression (2) will be described.

A graph 2000 in FIG. 20 indicates the above Expression (2). A vertical axis of the graph indicates $H(d_n)$, and a horizontal axis thereof indicates $d_n$. As illustrated in FIG. 20, in a case where a difference obtained by subtracting the cumulative travel distance in the nth step from the cumulative travel distance in the (n + 1)th step is larger than 0, the above Expression (2) is "1" indicating that the heel-strike point in the nth step is likely to be an actual heel-strike point. Further, in a case where the difference obtained by subtracting the cumulative travel distance in the nth step from the cumulative travel distance in the (n + 1)th step is equal to or less than 0, the above Expression (2) is "0" indicating that the heel-strike point in the nth step is not likely to be the actual heel-strike point. The information processing apparatus 100 calculates the first certainty factor by substituting the above Expression (2) for the following Expression (3).
[Expression 3]

$$E = \prod_{n=1}^{N} H(d_n) \quad \dots (3)$$

The symbol "E" represents the first certainty factor. The above Expression (3) indicates a multiplication value of $H(d_n)$ of each step. In a case where the cumulative travel distance is monotonically increased, the above Expression (3) is "1" indicating that the candidate group is likely to be a combination of actual heel-strike points. Further, in a case where the cumulative travel distance is not monotonically increased, the above Expression (3) is "0". With this, the information processing apparatus 100 can calculate the first certainty factor. Next, description of FIG. 21 will be made, and another specific example of calculating the first certainty factor will be described.

A graph 2100 in FIG. 21 associates the number of steps in a certain candidate group with a cumulative travel distance of the number of steps. A horizontal axis of the graph indicates the number of steps, and a vertical axis thereof indicates the cumulative travel distance. As illustrated in FIG. 21, the cumulative travel distances in the second and fourth steps are smaller than the cumulative travel distances in the first and third steps, respectively, in the certain candidate group, and the cumulative travel distance is not monotonically increased.

However, the patient may drag his/her leg due to illness or injury, and the patient may walk while moving the swing leg only up to a position existing at a predetermined distance behind the support leg. Therefore, when considering disease or injury, the walking movement of the patient has such a property that the landing position of the leg serving as the swing leg of the patient falls within a range from a start point existing at the second distance behind the landing position of the leg serving as the support leg of the patient to an end point existing at the first distance in front of the start point. In other words, in the walking movement of the patient, the cumulative travel distance in each step may not be monotonically increased.

Therefore, the information processing apparatus 100 also preferably calculates the first certainty factor of the candidate group illustrated as the graph in FIG. 21 so that the first certainty factor is not reduced. Specifically, the information processing apparatus 100 calculates the first certainty factor by using the following Expression (4) instead of the above Expression (2) .
[Expression 4]

$$H(d_n) = \begin{cases} 1(d_n \geq \varepsilon) \\ 0(d_n < \varepsilon) \end{cases} \quad \dots (4)$$

The symbol "$d_n$" represents a difference obtained by subtracting the cumulative travel distance in the nth step from the cumulative travel distance in the (n + 1)th step. The symbol "$\varepsilon$" corresponds to the second distance described above. The symbol "$H(d_n)$" represents a value indicating whether or not the heel-strike point in the nth step is likely to be an actual heel-strike point. In a case where $H(d_n)$ is 1, the heel-strike point is likely to be an actual heel-strike point. Herein, description of FIG. 22 will be made, and the above Expression (4) will be described.

A graph 2200 in FIG. 22 shows the above Expression (4). A vertical axis of the graph indicates $H(d_n)$, and a horizontal axis thereof indicates $d_n$. As illustrated in FIG. 22, in a case where a difference obtained by subtracting the cumulative travel distance in the nth step from the cumulative travel distance in the (n + 1)th step is larger than $-\varepsilon$, the above Expression (4) is "1" indicating that the heel-strike point in the nth step is likely to be an actual heel-strike point. Further, in a case where the difference obtained by subtracting the cumulative travel distance in the nth step from the cumulative travel distance in the (n + 1)th step is equal to or less than $-\varepsilon$, the above Expression (4) is "0" indicating that the heel-strike point in the nth step is not likely to be the actual heel-strike point. The information processing apparatus 100 calculates the first certainty factor by substituting the above Expression (4) for the above Expression (3) instead of the above Expression (2). Next, description of FIG. 23 will be made, and another specific example of calculating the first certainty factor will be described.

In FIG. 23, the information processing apparatus 100 can further calculate the first certainty factor also on the basis of information other than the degree of the increase in the cumulative travel distance in each step. The information processing apparatus 100 calculates the first certainty factor by using, for example, a difference between the cumulative travel distances of both the legs of the patient.

For example, in the walking movement of the patient, in a case where the same leg is the swing leg even if the steps are different, variation of how far the landing position of the leg moves forward from the leg serving as the support leg tends to be small. Therefore, the information processing apparatus 100 calculates the first certainty factor so that the first certainty factor is higher as variation in the difference between the cumulative travel distances of both the legs of the patient is decreased. The information processing apparatus 100 calculates the first certainty factor by using, for example, the following Expression (5) instead of the above Expression (3).
[Expression 5]

$$E = \frac{1}{V} \prod_{n=1}^{N} H(d_n) \quad \dots (5)$$

The symbol "V" represents a degree of variation. The symbol "V" is, for example, a coefficient of variation of the difference $d_n$ between the cumulative travel distances calculated for one leg. With this, the information processing apparatus 100 can improve accuracy of calculating the first certainty factor. Next, another specific example of calculating the first certainty factor will be described with reference to FIG. 24.

A graph 2400 in FIG. 24 associates the number of steps obtained in a case where the patient walks while turning with a cumulative travel distance of the number of steps. A horizontal axis of the graph indicates the number of steps, and a vertical axis thereof indicates the cumulative travel distance. In the example of FIG. 24, because the patient walks while turning, the cumulative travel distance in the fourth step is smaller than the cumulative travel distance in the third step, and the cumulative travel distance is not monotonically increased.

Thus, in a case where the patient walks while turning, the step length of the inner leg tends to be small. Therefore, the walking movement of the patient has such a property that the cumulative travel distance may not be monotonically increased in a case where the patient walks while turning. In other words, the walking movement of the patient has such a property that the landing position of the leg serving as the swing leg of the patient falls within a range from a start point existing at the second distance behind the landing position of the leg serving as the support leg of the patient to an end point existing at the first distance in front of the start point. Therefore, the information processing apparatus 100 calculates the first certainty factor by using the following Expression (6) instead of the above Expression (2).
[Expression 6]

$$H(d_n) = \begin{cases} 1(d_n \geq \varepsilon + \delta) \\ 0(d_n < \varepsilon + \delta) \end{cases} \quad \dots (6)$$

The symbol "$d_n$" represents a difference obtained by subtracting the cumulative travel distance in the nth step from the cumulative travel distance in the (n + 1)th step. The expression "$\varepsilon + \delta$" corresponds to the second distance described above. The symbol "$\delta$" represents a parameter corresponding to a degree to which the patient turns in the (n-1)th step. The symbol "$H(d_n)$ " represents a value indicating whether or not the heel-strike point in the nth step is likely to be an actual heel-strike point. In a case where $H(d_n)$ is 1, the heel-strike point is likely to be an actual heel-strike point. Herein, description of FIG. 25 will be made, and the above Expression (6) will be described.

A graph 2500 in FIG. 25 shows the above Expression (6). A vertical axis of the graph indicates $H(d_n)$, and a horizontal axis thereof indicates $d_n$. As illustrated in FIG. 25, in a case where a difference obtained by subtracting the cumulative travel distance in the nth step from the cumulative travel distance in the (n + 1)th step is larger than $-(\varepsilon + \delta)$, the above Expression (6) is "1" indicating that the heel-strike point in the nth step is likely to be an actual heel-strike point. Further, in a case where the difference obtained by subtracting the cumulative travel distance in the nth step from the cumulative travel distance in the (n + 1)th step is equal to or less than $-(\varepsilon + \delta)$, the above Expression (6) is "0" indicating that the heel-strike point in the nth step is not likely to be the actual heel-strike point. The information processing apparatus 100 calculates the first certainty factor by substituting the above Expression (6) for the above Expression (3) instead of the above Expression (2).

Further, the information processing apparatus 100 may combine the above-mentioned methods of calculating the first certainty factor. The information processing apparatus 100 calculates the first certainty factor by using, for example, the following Expressions (7) and (8).
[Expression 7]

$$E(\varepsilon, \delta, V) = \frac{1}{V} \prod_{n=1}^{N} H(d_n) \quad ...(7)$$

The left side "$E(\varepsilon, \delta, V)$" represents the first certainty factor. The symbol "V" represents a degree of variation. The symbol "V" is, for example, a coefficient of variation of the difference $d_n$ between the cumulative travel distances calculated for one leg.

[Expression 8]

$$H(d_n) = \begin{cases} 1(d_n \geq \varepsilon + \delta) \\ 0(d_n < \varepsilon + \delta) \end{cases} \quad ...(8)$$

The symbol "$d_n$" represents a difference obtained by subtracting the cumulative travel distance in the nth step from the cumulative travel distance in the (n + 1)th step. The expression "$\varepsilon + \delta$" corresponds to the second distance described above. The symbol "$\delta$" represents a parameter corresponding to a degree to which the patient turns in the (n-1)th step. With this, the information processing apparatus 100 can improve accuracy of calculating the first certainty factor.

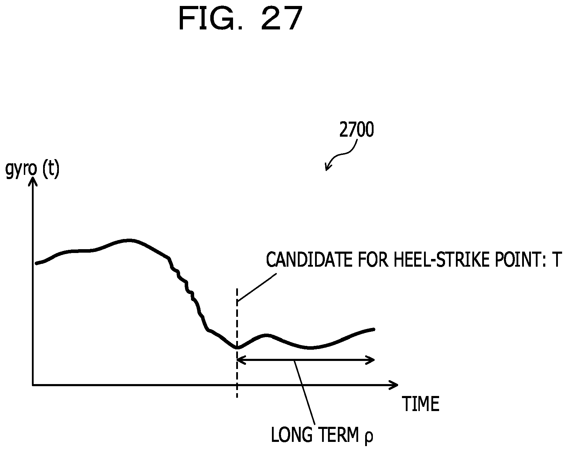

In addition, the information processing apparatus 100 may further calculate, in addition to the first certainty factor, a second certainty factor for evaluating likelihood of a combination of the heel-strike points in the candidate group also on the basis of information other than the cumulative travel distance. The other information is, for example, a feature value regarding the movement of each leg of the patient. Next, description of FIGS. 26 and 27 will be made, and an example of calculating the second certainty factor will be described.

In FIGS. 26 and 27, first, the information processing apparatus 100 calculates a plurality of feature values of the left leg and the right leg in order to calculate the second certainty factor.

The plurality of feature values include, for example, feature values indicating, for candidates for the heel-strike point, whether or not features corresponding to features of both the legs at the actual heel-strike points appear. The feature values are, for example, a short-term change amount $\alpha_1$ of the angular velocity and an absolute value $\alpha_3$ of the vertical acceleration of the leg serving as the swing leg in a candidate for the heel-strike point. Further, the feature values are a short-term change amount $\alpha_2$ of the angular velocity on the sagittal plane and an absolute value $\alpha_4$ of the vertical acceleration of the leg serving as the support leg in a candidate for the heel-strike point. The information processing apparatus 100 calculates the short-term change amount $\alpha_1$ by using, for example, the following Expression (9).

[Expression 9]

$$\alpha_1 = \frac{gyro(T + \tau) - gyro(T)}{\tau} \quad ...(9)$$

The symbol "$\tau$" represents a short time. The term "gyro(t)" represents angular velocity at a time t. The symbol "T" or "T + $\tau$" is substituted for t. The symbol "T" represents a candidate for the heel-strike point. Next, an example of calculating the short-term change amount $\alpha_1$ will be described with reference to FIG. 26.

In a graph 2600 of FIG. 26, a horizontal axis indicates time, and a vertical axis indicates angular velocity. As illustrated in FIG. 26, by using the above Expression (9), the information processing apparatus 100 calculates the short-term change amount $\alpha_1$ on the basis of the angular velocity in a period of $\tau$ after the heel-strike point. Similarly, the information processing apparatus 100 calculates the short-term change amount $\alpha_2$.

Further, the plurality of feature values include, for example, feature values indicating whether or not a feature has consistency before and after the candidate for the heel-strike point. The feature values are, for example, an absolute value $\beta_1$ and a long-term change amount $\beta_2$ of the angular velocity on the sagittal plane of the leg serving as the swing leg in the candidate for the heel-strike point. The information processing apparatus 100 calculates the long-term change amount $\beta_2$ by using, for example, the following Expression (10).

[Expression 10]

$$\beta_2 = \frac{gyro(T + \rho) - gyro(T)}{\rho} \quad ...(10)$$

The symbol "$\rho$" represents a long time and is longer than $\tau$. The term "gyro(t)" represents angular velocity at a time t. The symbol "T" or "T + $\tau$" is substituted for t. The symbol "T" represents the heel-strike point. Next, an example of calculating the long-term change amount $\beta_2$ will be described with reference to FIG. 27.

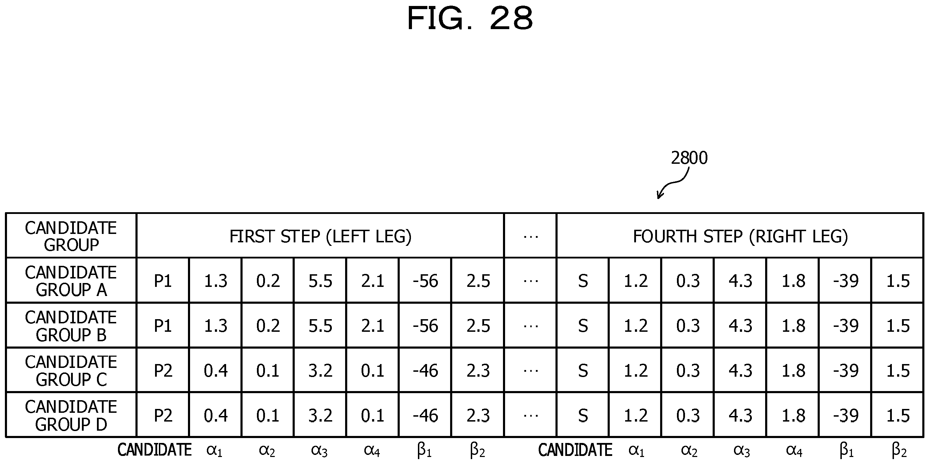

In a graph 2700 of FIG. 27, a horizontal axis indicates time, and a vertical axis indicates angular velocity. As illustrated in FIG. 27, by using the above Expression (10), the information processing apparatus 100 calculates the long-term variation $\beta_2$ on the basis of the angular velocity in a period of $\rho$ after the heel-strike point. Next, description of FIG. 28 will be made.

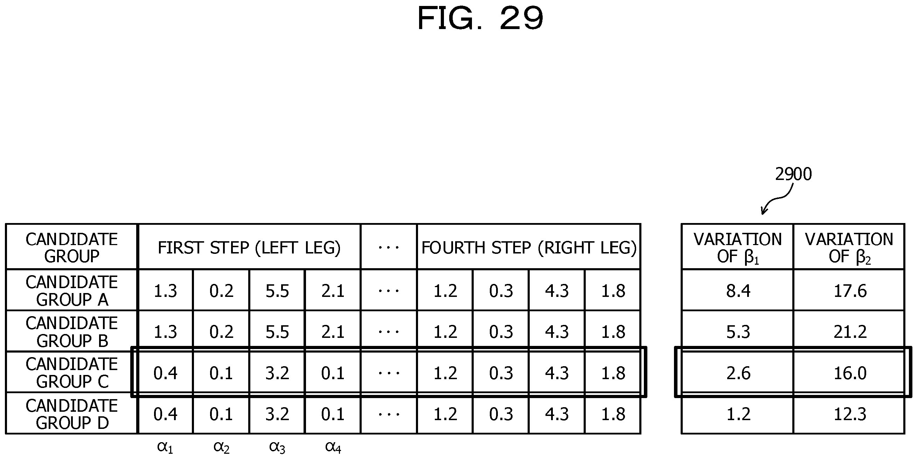

In FIG. 28, the information processing apparatus 100 calculates, for each step of each of the plurality of candidate groups, the above-mentioned feature values $\alpha_1$ to $\alpha_4$ and feature values $\beta_1$ and $\beta_2$. The information processing apparatus 100 stores the calculated result by using a feature value management table 2800. Next, description of FIG. 29 will be made.

In FIG. 29, the information processing apparatus 100 refers to the feature value management table 2800 and calculates the second certainty factor. For example, the information processing apparatus 100 determines that a set of candidate points in which the feature values $\alpha_1$ to $\alpha_4$ of both the legs are equal to or larger than a threshold in all steps and variation of the feature values $\beta_1$ and $\beta_2$ is equal to or less than a threshold is likely to be a combination of heel-strike points.

Therefore, the information processing apparatus 100 calculates the second certainty factor so that the second certainty factor is higher in a case where the feature values $\alpha_1$ to $\alpha_4$ of both the legs are equal to or larger than the threshold in all steps and the variation of the feature values $\beta_1$ and $\beta_2$ is equal to or less than the threshold. The information processing apparatus 100 calculates the second certainty factor by using, for example, the following Expressions (11) to (14).

[Expression 11]

$$E' = e_1 \times e_2 \quad \ldots (11)$$

The symbol "E'" represents the second certainty factor. The symbols "$e_1$" and "$e_2$" represent a certainty factor regarding $\alpha_i{}^n$ and a certainty factor regarding $\beta_j{}^n$ defined by the following Expressions (12) and (13), respectively.

[Expression 12]

$$e_1 = \prod_{i=1}^{I} \prod_{n=1}^{N} H(\alpha_i^n) \quad \ldots (12)$$

The symbol "$\alpha_i{}^n$" represents a feature value in the nth step and is, for example, the feature values $\alpha_1$ to $\alpha_4$. The term "H()" is defined by the following Expression (14).

[Expression 13]

$$e_2 = \prod_{j=1}^{J} H\left(var(\beta_j^n)\right) \quad \ldots (13)$$

The symbol "$\beta_j{}^n$" represents a feature value in the nth step and is, for example, the feature values $\beta_1$ and $\beta_2$. The term "var($\beta_j{}^n$)" represents a variance value of $\beta_j{}^n$. The term "H()" is defined in the same way as the following Expression (14).

[Expression 14]

$$H(\alpha_i^n) = \begin{cases} 1(\alpha_i^n \geq th_i) \\ 0(\alpha_i^n < th_i) \end{cases} \quad \ldots (14)$$

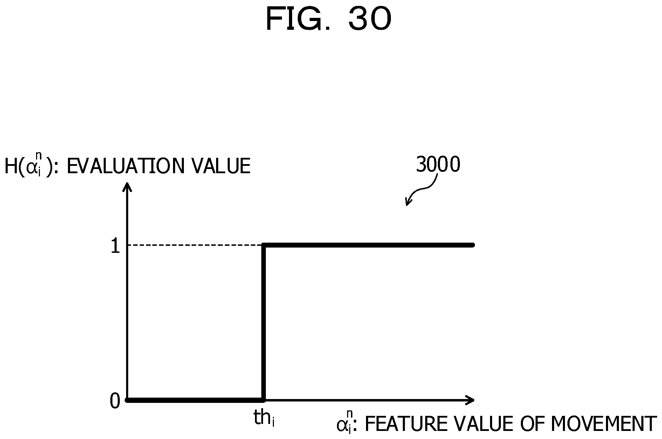

The symbol "thi" represents a threshold of the feature values $\alpha_1$ to $\alpha_4$. Herein, description of FIG. 30 will be made, and the above Expression (14) will be described.

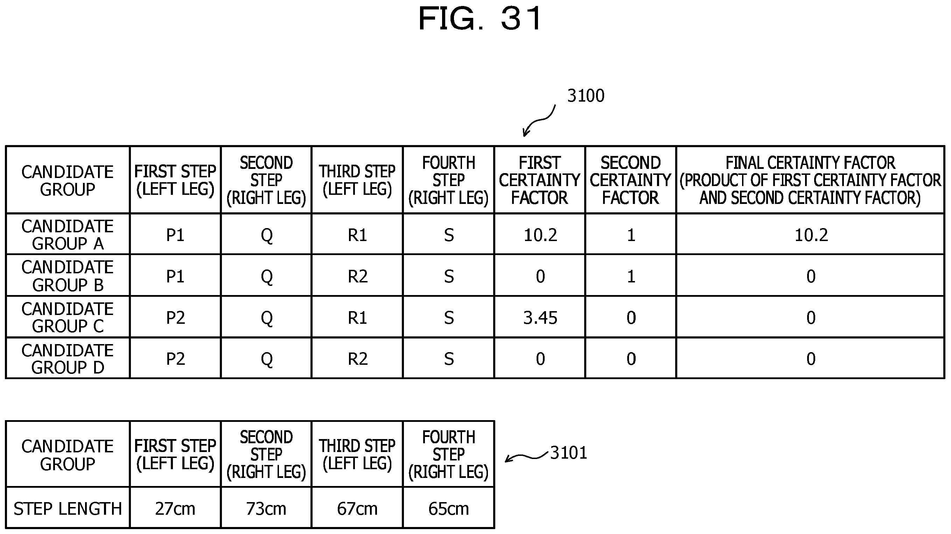

A graph 3000 in FIG. 30 shows the above Expression (14). A vertical axis of the graph indicates H($\alpha_i{}^n$), and a horizontal axis thereof indicates $\alpha_i{}^n$. As illustrated in FIG. 30, in a case where the feature values $\alpha_1$ to $\alpha_4$ are larger than the threshold $th_i$, the above Expression (14) is "1" indicating that the candidate group is likely to be a combination of actual heel-strike points. Further, in a case where the feature values $\alpha_1$ to $\alpha_4$ are equal to or smaller than the threshold $th_i$, the above Expression (14) is "0" indicating that the candidate group is not likely to be a combination of actual heel-strike points. Next, description of FIG. 31 will be made.

In FIG. 31, the information processing apparatus 100 stores the first certainty factor and the second certainty factor calculated for each candidate group in association with the candidate group by using a final certainty factor management table 3100. Then, the information processing apparatus 100 selects a candidate group having a relatively large first certainty factor and second certainty factor as a likely candidate group of a combination of actual heel-strike points.

For example, the information processing apparatus 100 calculates a value obtained by multiplying the first certainty factor and the second certainty factor as a final certainty factor and stores the calculated value by using the final certainty factor management table 3100. Then, the information processing apparatus 100 selects a candidate group having the maximum final certainty factor. With this, even in a case where one heel-strike point is not determined, the information processing apparatus 100 can select a likely candidate group of a combination of actual heel-strike points from a plurality of candidate groups.

When the information processing apparatus 100 selects the candidate group, the information processing apparatus 100 refers to the step length management table 1600 and outputs the step length calculated for the selected candidate group. The information processing apparatus 100 may store the step length calculated for the selected candidate group by using a final result table 3101. With this, the information processing apparatus 100 can improve accuracy of calculating the step length of the patient.

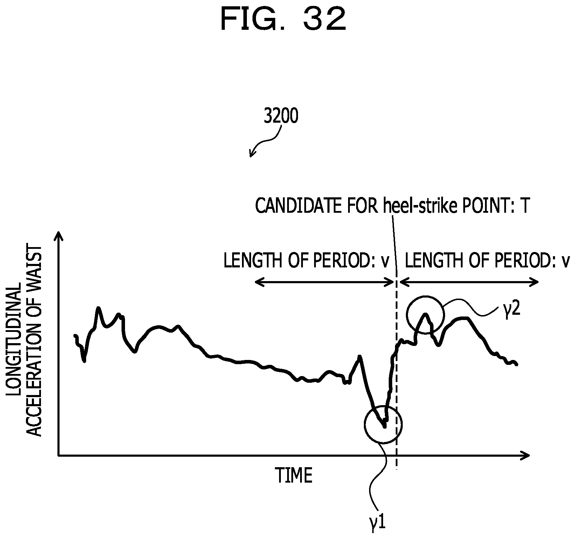

Further, the information processing apparatus 100 similarly selects a candidate group for each of the eleven partial periods divided in FIG. 12, thereby improving the accuracy of calculating the step length. Next, description of FIG. 32 will be made, and another specific example of calculating the second certainty factor will be described.

In FIG. 32, the information processing apparatus 100 calculates the second certainty factor on the basis of the plurality of feature values of the left leg and the right leg and then corrects the second certainty factor by using a plurality of feature values regarding the movement of the waist.

The plurality of feature values regarding the movement of the waist includes, for example, a feature value indicating whether or not a feature in which an upper part of the body of the patient is decelerated at the heel-strike point appears and a feature value indicating whether or not a feature in which the upper part of the body is accelerated after the heel-strike point appears. The feature values are, for example, the minimum value $\gamma 1$ of the longitudinal acceleration of the waist immediately before the heel-strike point and the maximum value $\gamma 2$ of the longitudinal acceleration of the waist immediately after the heel-strike point.

A graph 3200 in FIG. 32 shows acceleration data of the longitudinal acceleration of the waist. A horizontal axis of the graph indicates time, and a vertical axis thereof indicates the longitudinal acceleration.

Based on the acceleration data of the longitudinal acceleration of the waist, the information processing apparatus 100 calculates, as the minimum value $\gamma 1$, the minimum value of the longitudinal acceleration of the waist in a period [T-$\nu$, T] immediately before the candidate T for the heel-strike point. Further, the information processing apparatus 100 calculates, as the maximum value $\gamma 2$, the maximum value of the longitudinal acceleration of the waist in a period [T, T + $\nu$] immediately before the candidate for the heel-strike point.

Then, the information processing apparatus 100 calculates the second certainty factor so that the second certainty factor is higher in a case where the feature values $\gamma 1$ and $\gamma 2$ are equal to or larger than thresholds. For example, the information processing apparatus 100 calculates the second certainty factor by using the following Expressions (15) and (16) on the basis of the minimum value $\gamma 1$ and the maximum value $\gamma 2$.

[Expression 15]

$$E' = e_1 \times e_2 \times e_3 \quad \dots (15)$$

The symbol "E'" represents the second certainty factor. The symbols "$e_1$" and "$e_2$" represent a certainty factor regarding $\alpha_i^n$ and a certainty factor regarding $\beta_j^n$ defined by the above Expressions (12) and (13), respectively. The symbol "$e_3$" represents a certainty factor regarding $\gamma_k^n$ defined by the following Expression (16).

[Expression 16]

$$e_3 = \prod_{k=1}^{K} \prod_{n=1}^{N} H(\gamma_k^n) \quad \dots (16)$$

The symbol "$\gamma_k^n$" indicates a feature value in the nth step and is, for example, the feature values $\gamma 1$ and $\gamma 2$. The term "H()" is defined in the same way as the above Expression (14). With this, in a case where the feature values $\gamma 1$ and $\gamma 2$ are equal to or larger than the thresholds, the certainty factor $e_3$ is set to 1, and, in a case where one of the feature values $\gamma 1$ and $\gamma 2$ is less than the threshold, the certainty factor $e_3$ is set to 0.

In this way, the information processing apparatus 100 can calculate the certainty factor $e_3$ corresponding to whether or not the feature in which the upper part of the body of the patient is decelerated at the heel-strike point appears and whether or not the feature in which the upper part of the body is accelerated after the heel-strike point appears. With this, the information processing apparatus 100 can improve accuracy of calculating the second certainty factor. Next, description of FIG. 33 will be made, and another specific example of calculating the second certainty factor will be described.

In FIG. 33, the information processing apparatus 100 determines a reference for calculating the second certainty factor on the basis of the reaction force data and calculates the second certainty factor on the basis of the feature value $\alpha_i^n$, the feature value $\beta_j^n$, the feature value $\gamma_k^n$, and the like.

As illustrated in FIG. 33, the information processing apparatus 100 receives reaction force data including reaction force at the heel-strike point from the measuring instrument 201 serving as the reaction force sensor device. The information processing apparatus 100 specifies the heel-strike point on the basis of the received reaction force data.

Based on the gyro data and the acceleration data regarding the left leg, the right leg, and the waist of the patient, the information processing apparatus 100 stores, for the specified heel-strike point, a feature value $\alpha_i^{n'}$, a feature value $\beta_j^{n'}$, a feature value $\gamma_k^{n'}$, and the like as references of the feature values at the heel-strike point.

Then, the information processing apparatus 100 calculates the feature value $\alpha_i^n$, the feature value $\beta_j^n$, and the feature value $\gamma_k^n$ for each candidate for the heel-strike point in each candidate group and calculates degrees of similarity to the feature value $\alpha_i^{n'}$, the feature value $\beta_j^{n'}$, and the feature value $\gamma_k^{n'}$ serving as the references. Then, the information processing apparatus 100 uses the degrees of similarity as the certainty factors $e_1$, $e_2$, and $e_3$ and calculates the second certainty factor by using the above Expression (15). The certainty factors $e_1$, $e_2$, and $e_3$ are, for example, norms.

In addition, the information processing apparatus 100 may further calculate, in addition to the first and second certainty factors, a third certainty factor for evaluating likelihood of a combination of the heel-strike points in the candidate group. Next, description of FIG. 34 will be made, and a specific example of calculating the third certainty factor will be described.

In FIG. 34, the information processing apparatus 100 receives a travel distance 3400 of the patient based on the GPS coordinates or the like from the sensor device. Herein, the travel distance 3400 of the patient based on the GPS coordinates or the like tends to have a value close to the cumulative travel distance in the final step of a likely candidate group of a combination of actual heel-strike points.

Therefore, the information processing apparatus 100 calculates the third certainty factor on the basis of the error $\varepsilon$ between the cumulative travel distance in the final step of each of the plurality of candidate groups and the received travel distance 3400. The information processing apparatus 100 calculates the third certainty factor by using, for example, the following Expression (17).

[Expression 17]

$$E(\varepsilon) = \frac{1}{1 + exp(a_1\varepsilon - a_2)} \quad \dots (17)$$

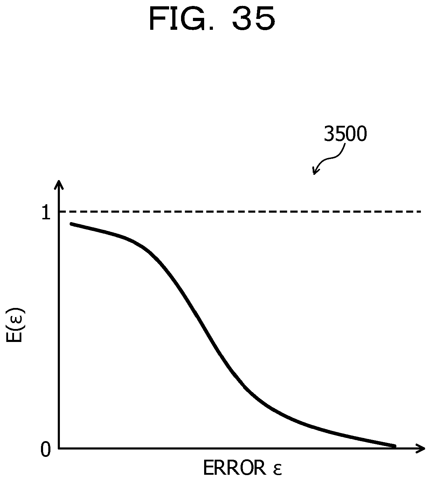

Herein, description of FIG. 35 will be made, and the above Expression (17) will be described.

A graph 3500 in FIG. 35 shows the above Expression (17). A vertical axis of the graph indicates $E(\varepsilon)$, and a horizontal axis thereof indicates $\varepsilon$. The above Expression (17) is a logistic curve. The symbols "a1" and "a2" represent parameters for determining a shape of a function of the above Expression (17) and are set in advance. The symbol "a1" is positive. With this, the information processing apparatus 100 can calculate the third certainty factor.

When the information processing apparatus 100 calculates the third certainty factor, the information processing apparatus 100 calculates, for example, a value obtained by multiplying the first certainty factor, the second certainty factor, and the third certainty factor as the final certainty factor. Then, the information processing apparatus 100 selects one of the plurality of candidate groups on the basis of the final certainty factor. With this, the information processing apparatus 100 can improve accuracy of selecting a candidate group.

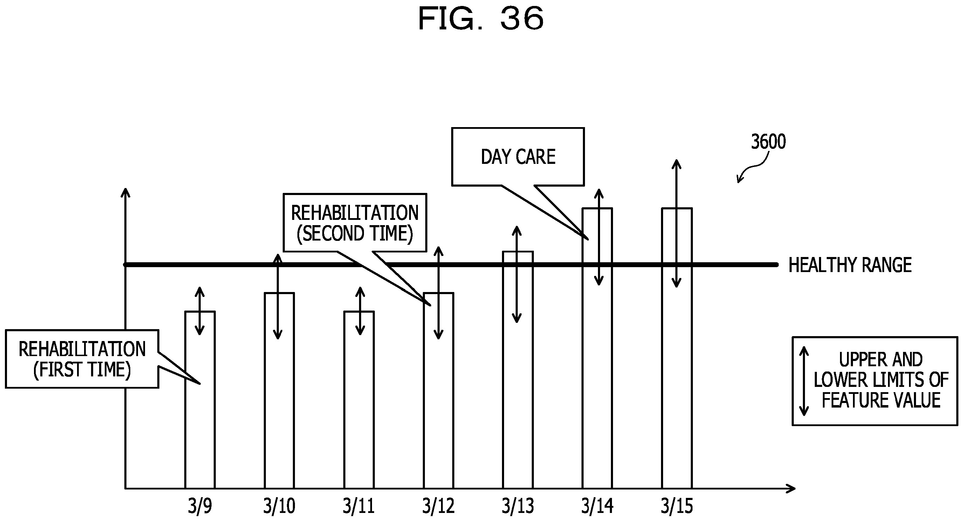

In FIG. 36, the information processing apparatus 100 specifies a tendency of the step length of the patient on the basis of the calculated step length and outputs the tendency. For example, the information processing apparatus 100 accepts specification of a target date range and reads the step length within the date range. Then, in order to grasp transition of the daily way of walking of the patient, the information processing apparatus 100 calculates a statistical value of the step length at each date and displays the statistical value as illustrated in a graph 3600.

Herein, the information processing apparatus 100 may display, on the graph 3600, a range of reference values obtained in a case where the patient is healthy. Further, the information processing apparatus 100 may display, on the graph 3600, a timing at which a reference event regarding the patient is performed, such as a future medical care for the patient. In addition, the information processing apparatus 100 may display, on the graph 3600, a range from an upper limit to a lower limit of the step length of the patient for each date.

(One Example of Overall Processing Procedure)

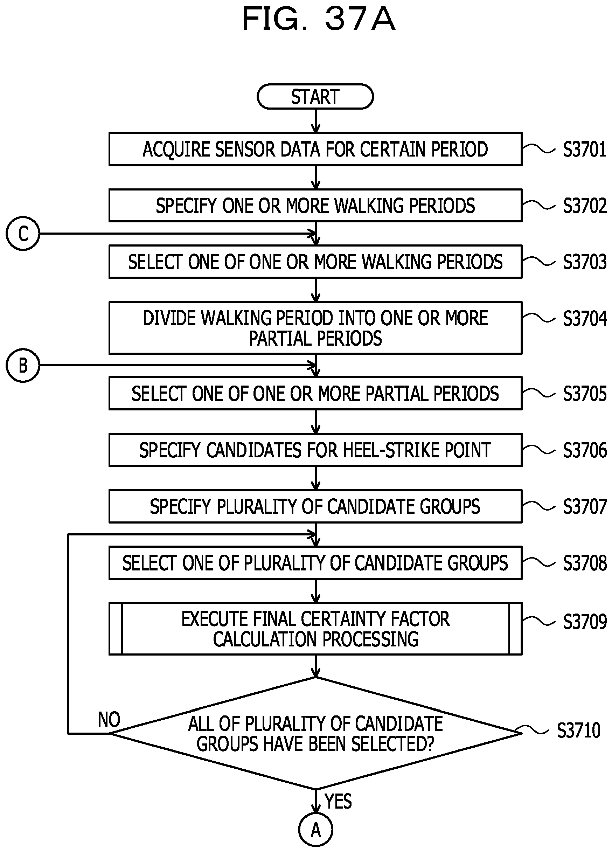

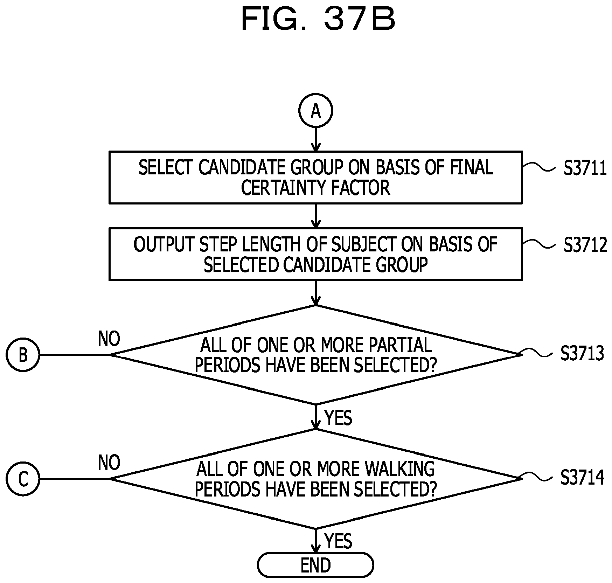

Next, an example of an overall processing procedure executed by the information processing apparatus 100 will be described with reference to FIG. 37.

FIG. 37 is a flowchart illustrating an example of an overall processing procedure. First, the information processing apparatus 100 acquires sensor data of each leg of the patient or each leg and the waist of the patient for a certain period from the measuring instrument 201 (Step S3701) .

Then, the information processing apparatus 100 specifies one or more walking periods on the basis of the acquired sensor data (Step S3702). Then, the information processing apparatus 100 selects the one or more specified walking periods (Step S3703).

Then, the information processing apparatus 100 divides the selected walking period into one or more partial periods (Step S3704). Then, the information processing apparatus 100 selects one of the one or more divided partial periods (Step S3705).

Then, the information processing apparatus 100 specifies candidates for the heel-strike point in the selected partial period (Step S3706). Then, the information processing apparatus 100 specifies a plurality of candidate groups obtained by combining the specified candidates (Step S3707).

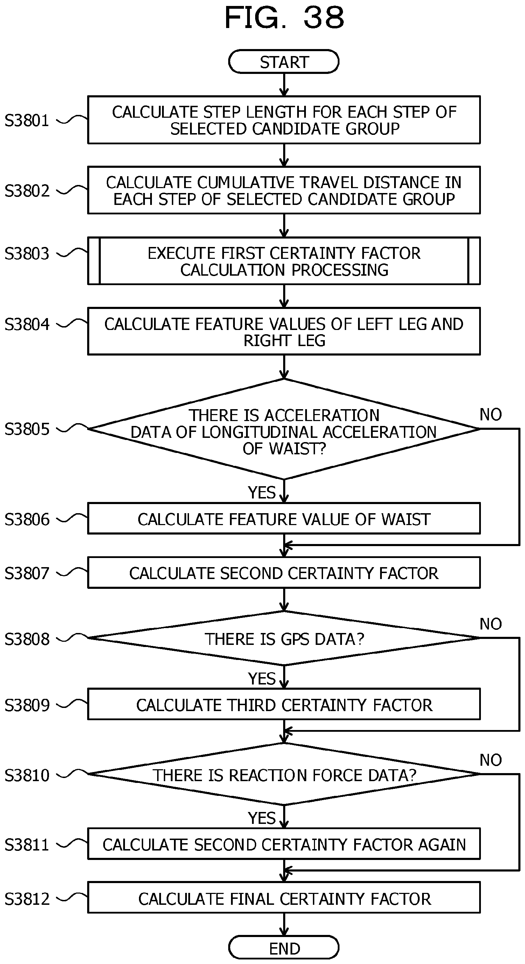

Then, the information processing apparatus 100 selects one of the plurality of specified candidate groups (Step S3708). Then, the information processing apparatus 100 executes, for the selected candidate group, final certainty factor calculation processing described below with reference to FIG. 38 (Step S3709).

Then, the information processing apparatus 100 determines whether or not all of the plurality of specified candidate groups have been selected (Step S3710). Herein, in a case where not all of the plurality of specified candidate groups have been selected (Step S3710: No), the information processing apparatus 100 returns to the processing in Step S3708.

Meanwhile, in a case where all of the plurality of specified candidate groups have been selected (Step S3710: Yes), the information processing apparatus 100 selects one of the candidate groups on the basis of the calculated final certainty factor (Step S3711). Then, the information processing apparatus 100 calculates the step length of the subject on the basis of the selected candidate group and outputs the calculated step length (Step S3712) .

Then, the information processing apparatus 100 determines whether or not all of the one or more divided partial periods have been selected (Step S3713). Herein, in a case where not all of the divided partial periods have been selected (Step S3713: No), the information processing apparatus 100 returns to the processing in Step S3705.

Meanwhile, in a case where all of the divided partial periods have been selected (Step S3713: Yes), the information processing apparatus 100 determines whether or not all of the one or more specified walking periods have been selected (Step S3714). Herein, in a case where not all of the one or more specified walking periods have been selected (Step S3714: No), the information processing apparatus 100 returns to the processing in Step S3703.

Meanwhile, in a case where all of the one or more specified walking periods have been selected (Step S3714: Yes), the information processing apparatus 100 terminates the overall processing.

(One Example of Final Certainty Factor Calculation Processing Procedure)

Next, an example of a final certainty factor calculation processing procedure executed by the information processing apparatus 100 will be described with reference to FIG. 38.

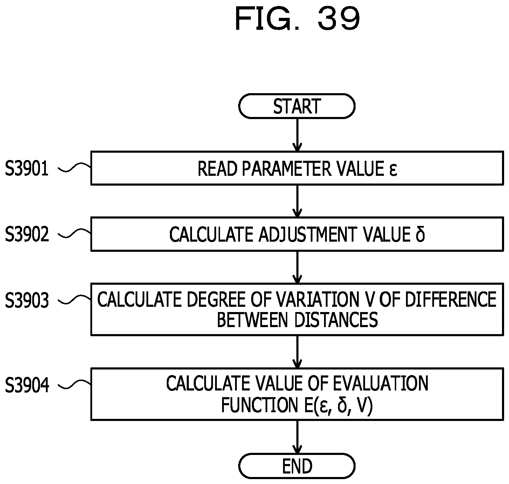

FIG. 38 is a flowchart illustrating an example of a final certainty factor calculation processing procedure. In FIG. 38, first, the information processing apparatus 100 calculates the step length for each step of the selected candidate group (Step S3801). Then, the information processing apparatus 100 calculates the cumulative travel distance in each step of the selected candidate group (Step S3802). Then, the information processing apparatus 100 executes first certainty factor calculation processing described below with reference to FIG. 39 (Step S3803).

Then, the information processing apparatus 100 calculates feature values of the left leg and the right leg (Step S3804). Then, the information processing apparatus 100 determines whether or not there is acceleration data of the longitudinal acceleration of the waist (Step S3805). Herein, in a case where there is no acceleration data (Step S3805: No), the information processing apparatus 100 proceeds to the processing in Step S3807.

Meanwhile, in a case where there is acceleration data (Step S3805: Yes), the information processing apparatus 100 calculates a feature value of the waist (Step S3806) . Then, the information processing apparatus 100 proceeds to the processing in Step S3807.

Then, the information processing apparatus 100 calculates the second certainty factor (Step S3807). Then, the information processing apparatus 100 determines whether or not there is GPS data (Step S3808) . Herein, in a case where there is no GPS data (Step S3808: No), the information processing apparatus 100 proceeds to the processing in Step S3810.

Meanwhile, in a case where there is GPS data (Step S3808: Yes), the information processing apparatus 100 calculates the third certainty factor (Step S3809). Then, the information processing apparatus 100 proceeds to the processing in Step S3810.

Then, the information processing apparatus 100 determines whether or not there is reaction force data (Step S3810). Herein, in a case where there is no reaction force data (Step S3810: No), the information processing apparatus 100 proceeds to the processing in Step S3812.

Meanwhile, in a case where there is reaction force data (Step S3810: Yes), the information processing apparatus 100 calculates the second certainty factor again (Step S3811). Then, the information processing apparatus 100 proceeds to the processing in Step S3812.

Then, the information processing apparatus 100 calculates the final certainty factor (Step S3812). Then, the information processing apparatus 100 terminates the final certainty factor calculation processing.

(One Example of First Certainty Factor Calculation Processing Procedure)

Next, an example of a first certainty factor calculation processing procedure executed by the information processing apparatus 100 will be described with reference to FIG. 39.

FIG. 39 is a flowchart illustrating an example of a first certainty factor calculation processing procedure. In FIG. 39, first, the information processing apparatus 100 reads a parameter value ε (Step S3901). Then, the information processing apparatus 100 calculates an adjustment value δ (Step S3902).

Then, the information processing apparatus 100 calculates a degree of variation V of a difference between distances (Step S3903). Then, the information processing apparatus 100 then calculates a value of an evaluation function $E(ε, δ, V)$ (Step S3904). Thereafter, the information processing apparatus 100 terminates the first certainty factor calculation processing.

As described above, the information processing apparatus 100 can specify a plurality of candidate groups obtained by selecting, for each step in the walking period of the subject, one of one or more candidates for the point of time corresponding to landing of the leg that serving as the swing leg of the subject and combining the selected candidates. The information processing apparatus 100 can calculate, for each candidate group, the cumulative travel distance of the leg serving as the swing leg of the subject for each step in the walking period. Based on the cumulative travel distance, the information processing apparatus 100 can evaluate, for each candidate group, a degree of appearance of the relationship in which the landing position of the leg serving as the swing leg of the subject falls within a predetermined range of the landing position of the leg serving as the support leg of the subject in the walking period. The information processing apparatus 100 can select one of the plurality of candidate groups on the basis of the evaluation result. With this, the information processing apparatus 100 can select a likely candidate group of a combination of the points of time at which the leg of the subject lands and can therefore improve accuracy of calculating the feature value of the subject such as the step length.

The information processing apparatus 100 can, for each candidate group, determine whether or not the relationship has appeared for each step in the walking period and can calculate an evaluation value of the candidate group so that the evaluation value of the candidate group is increased as the number of times of appearance of the relationship is larger. The information processing apparatus 100 can select one of the plurality of candidate groups on the basis of the calculated evaluation value of each candidate group. With this, the information processing apparatus 100 can select, from the plurality of candidate groups, the most likely candidate group of a combination of the points of time at which the leg of the subject lands.

The information processing apparatus 100 can set, as a predetermined range, a range from a start point that is the landing position of the leg serving as the support leg of the subject to an end point that is a position existing at the first distance in front of the start point. With this, the information processing apparatus 100 can select a likely candidate group of a combination of points of the time at which the leg of the subject lands corresponding to a case where, for example, the subject normally walks.

The information processing apparatus 100 can set, as the predetermined range, a range from a start point that is a position existing at the second distance behind the landing position of the leg serving as the support leg of the subject to an end point that is a position existing at the first distance in front of the start point. With this, the information processing apparatus 100 can select a likely candidate group of a combination of points of the time at which the leg of the subject lands corresponding to a case where, for example, the subject walks while dragging his/her leg.

For each candidate group, the information processing apparatus 100 can set, for each step in the walking period, the second distance according to a rotation direction and a degree of rotation of the leg serving as the swing leg of the subject and determine whether or not the relationship has appeared. With this, the information processing apparatus 100 can select a likely candidate group of a combination of points of the time at which the leg of the subject lands corresponding to a case where, for example, the subject turns.

For each candidate group, the information processing apparatus 100 can calculate the evaluation value of the candidate group so that the evaluation value of the candidate group is increased as a difference between the cumulative

# EP 3 649 939 B1

travel distances of both the legs of the subject is constant in the walking period. With this, the information processing apparatus 100 can improve accuracy of selecting a likely candidate group of a combination of the points of time at which the leg of the subject lands.

The information processing apparatus 100 can calculate, for each candidate group, a predetermined feature value for each candidate on the basis of measurement information regarding the movement of the waist of the subject and select one of the plurality of candidate groups on the basis of the calculated predetermined feature value of each candidate. With this, the information processing apparatus 100 can improve accuracy of selecting a likely candidate group of a combination of the points of time at which the leg of the subject lands.

The information processing apparatus 100 can calculate, for each candidate group, a predetermined feature value for each candidate on the basis of measurement information regarding reaction force from the ground to the subject and select one of the plurality of candidate groups on the basis of the calculated predetermined feature value of each candidate. With this, the information processing apparatus 100 can improve accuracy of selecting a likely candidate group of a combination of the points of time at which the leg of the subject lands.

The information processing apparatus 100 can select, for each candidate group, one of the plurality of candidate groups on the basis of a difference between the cumulative travel distance of the leg serving as the swing leg of the subject in the final step in the walking period and the acquired travel distance of the subject. With this, the information processing apparatus 100 can improve accuracy of selecting a likely candidate group of a combination of the points of time at which the leg of the subject lands.

The information processing apparatus 100 can set, as the walking period, a period in which walking movement is performed by the subject and the number of steps of the subject is less than a predetermined number of steps. With this, the information processing apparatus 100 can reduce throughput for selecting a likely candidate group of a combination of the points of time at which the leg of the subject lands.

Note that the information processing method described in this embodiment can be implemented by executing a prepared program on a computer such as a personal computer or a workstation. This information processing program is recorded on a computer-readable recording medium such as a hard disk, flexible disk, compact disk read only memory (CD-ROM), magneto-optical disk (MO), or digital versatile disc (DVD), and is read from the recording medium to be executed by the computer. In addition, this information processing program may be distributed via a network such as the Internet.

REFERENCE SIGNS LIST

100 information processing apparatus

110 schematic diagram

200 information processing system

201 measuring instrument

210 network

300, 400 bus

301, 401 CPU

302, 402 memory

303, 403 network I/F

304 recording medium I/F

305 recording medium

404 sensor unit

405 timer unit

23

500 storage unit

501 acquisition unit

502 specification unit

503 calculation unit

504 evaluation unit

505 selection unit

506 output unit

1000, 1002 gyro data

1001, 1003 acceleration data

1100 walking period management table

1301 to 1303 point of time

1400 candidate group management table

1600 step length management table

1700 cumulative travel distance management table

1800 first certainty factor management table

1900, 2000, 2100, 2200, 2400, 2500, 2600, 2700, 3000, 3200, 3500, 3600 graph

2800 feature value management table

3100 final certainty factor management table

3101 final result table

3400 travel distance

**Claims**

1.  An information processing apparatus (100) comprising
    a control unit (401) configured to:

    acquire measurement information regarding movement of each leg of a subject;
    specify, for each step in a walking period of the subject, one or more candidates for a point of time corresponding to landing of a leg serving as a swing leg of the subject on the basis of the acquired measurement information;
    specify a plurality of candidate groups obtained by selecting one of the one or more candidates specified for each step in the walking period and combining the selected candidates;
    calculate, for each candidate group of the plurality of specified candidate groups, a step length of each step in the walking period and calculate, for each step in the walking period, a cumulative travel distance of the leg serving as the swing leg of the subject on the basis of the calculated step length of each step in the walking period;
    evaluate, for each candidate group, a degree of appearance of a relationship on the basis of the cumulative travel distance calculated for each step in the walking period, the relationship being a relationship in which a landing position of the leg serving as the swing leg of the subject falls within a predetermined range of a landing position of a leg serving as a support leg of the subject in the walking period;

select one of the plurality of candidate groups on the basis of a result of the evaluation; and
output the selected one of the plurality of candidate groups.

2. The information processing apparatus (100) according to claim 1, wherein

the control unit (401) is configured to
determine, for each candidate group, whether or not the relationship appears in each step of the walking period and calculate an evaluation value of the candidate group so that the evaluation value of the candidate group is increased as the number of times of appearance of the relationship is increased, and
select one of the plurality of candidate groups on the basis of the calculated evaluation value of each candidate group.

3. The information processing apparatus (100) according to claim 2, wherein
the predetermined range is a range from a start point that is the landing position of the leg serving the support leg of the subject to an end point that is a position existing at a first distance in front of the start point.

4. The information processing apparatus (100) according to claim 2, wherein
the predetermined range is a range from a start point that is a position existing at a second distance behind the landing position of the leg serving as the support leg of the subject to an end point that is a position existing at a first distance in front of the start point.

5. The information processing apparatus (100) according to claim 4, wherein
for each candidate group, the control unit (401) is configured to set, for each step in the walking period, the second distance according to a rotation direction and a degree of rotation of the leg serving as the swing leg of the subject and determine whether or not the relationship appears.

6. The information processing apparatus (100) according to any one of claims 2 to 5, wherein
for each candidate group, the control unit (401) is configured to calculate the evaluation value of the candidate group so that the evaluation value of the candidate group is increased as a difference between the cumulative travel distances of both the legs of the subject is constant in the walking period.

7. The information processing apparatus (100) according to any one of claims 1 to 6, wherein

the control unit (401) is configured to
acquire measurement information regarding movement of a waist of the subject, and
calculate, for each candidate group, a predetermined feature value for each candidate on the basis of the measurement information regarding the movement of the waist or each leg of the subject, and select one of the plurality of candidate groups on the basis of the calculated predetermined feature value of each candidate.

8. The information processing apparatus (100) according to any one of claims 1 to 7, wherein

the control unit (401) is configured to
acquire measurement information regarding reaction force from the ground to the subject, and
calculate, for each candidate group, a predetermined feature value for each candidate on the basis of the measurement information regarding the reaction force and the measurement information regarding the movement of each leg of the subject, and select one of the plurality of candidate groups on the basis of the calculated predetermined feature value of each candidate.

9. The information processing apparatus (100) according to any one of claims 1 to 8, wherein

the control unit (401) is configured to
acquire a travel distance of the subject, and
select, for each candidate group, one of the plurality of candidate groups on the basis of a difference between the cumulative travel distance of the leg serving as the swing leg of the subject in a final step in the walking period and the travel distance of the subject in the walking period.

10. The information processing apparatus (100) according to any one of claims 1 to 9, wherein
a period in which walking movement is performed by the subject and the number of steps of the subject is less than a predetermined number of steps is set as the walking period.

**11.** The information processing apparatus (100) according to any one of claims 1 to 10, wherein

the control unit (401) is configured to
specify the step length of the subject for each step in the walking period on the basis of the selected one of the candidate groups, and
output the specified step length.

**12.** An information processing system (200) comprising:

a measuring instrument (201); and
an information processing apparatus (100) according to any of claims 1 to 11 that can communicate with the measuring instrument (201), wherein
the measuring instrument is configured to generate the measurement information, and
the information processing apparatus (100) is configured to acquire the measurement information from the measuring instrument (201).

**13.** An information processing method for causing a computer to execute processing of:

acquiring measurement information regarding movement of each leg of a subject;
specifying, for each step in a walking period of the subject, one or more candidates for a point of time corresponding to landing of a leg serving as a swing leg of the subject on the basis of the acquired measurement information;
specifying a plurality of candidate groups obtained by selecting one of the one or more candidates specified for each step in the walking period and combining the selected candidates;
calculating, for each candidate group of the plurality of specified candidate groups, a step length of each step in the walking period and calculating, for each step in the walking period, a cumulative travel distance of the leg serving as the swing leg of the subject on the basis of the calculated step length of each step in the walking period;
evaluating, for each candidate group, a degree of appearance of a relationship on the basis of the cumulative travel distance calculated for each step in the walking period, the relationship being a relationship in which a landing position of the leg serving as the swing leg of the subject falls within a predetermined range of a landing position of a leg serving as a support leg of the subject in the walking period;
selecting one of the plurality of candidate groups on the basis of a result of the evaluation; and
outputting the selected one of the plurality of candidate groups.

**Patentansprüche**

**1.** Informationsverarbeitungsvorrichtung (100), umfassend
eine Steuereinheit (401), die ausgelegt ist zum:

Erfassen von Messinformationen bezüglich einer Bewegung jedes Beins einer Zielperson;
Spezifizieren, für jeden Schritt in einer Gehperiode der Zielperson, eines oder mehrerer Kandidaten für einen Zeitpunkt, der einer Landung eines Beins entspricht, das als Spielbein der Zielperson dient, basierend auf den erfassten Messinformationen;
Spezifizieren mehrerer Kandidatengruppen, die durch Auswählen eines der einen oder mehreren für jeden Schritt in der Gehperiode spezifizierten Kandidaten erhalten werden, und Kombinieren der ausgewählten Kandidaten;
Berechnen, für jede Kandidatengruppe aus den mehreren spezifizierten Kandidatengruppen, einer Schrittlänge für jeden Schritt in der Gehperiode, und Berechnen, für jeden Schritt in der Gehperiode, einer kumulativen Wegstrecke des Beins, das als Spielbein der Zielperson dient, basierend auf der berechneten Schrittlänge jedes Schritts in der Gehperiode;
Bewerten, für jede Kandidatengruppe, eines Grades eines Erscheinens einer Beziehung basierend auf der kumulierten Wegstrecke, die für jeden Schritt in der Gehperiode berechnet wurde, wobei die Beziehung eine Beziehung ist, in der eine Landeposition des Beins, das als Spielbein der Zielperson dient, in einen vorbestimmten Bereich einer Landeposition eines Beins fällt, das während der Gehperiode als Stützbein der Zielperson dient;

Auswählen einer der mehreren Kandidatengruppen basierend auf einem Ergebnis der Bewertung; und
Ausgeben der ausgewählten der mehreren Kandidatengruppen.

2. Informationsverarbeitungsvorrichtung (100) nach Anspruch 1, wobei

die Steuereinheit (401) ausgelegt ist zum
Bestimmen, für jede Kandidatengruppe, ob die Beziehung in jedem Schritt der Gehperiode erscheint oder nicht, und Berechnen eines Bewertungswerts der Kandidatengruppe, so dass der Bewertungswert der Kandidatengruppe erhöht wird, wenn die Häufigkeit einer Erscheinung der Beziehung erhöht wird, und
Auswählen einer der mehreren Kandidatengruppen basierend auf dem berechneten Bewertungswert jeder Kandidatengruppe.

3. Informationsverarbeitungsvorrichtung (100) nach Anspruch 2, wobei
der vorbestimmte Bereich ein Bereich von einem Startpunkt, der die Landeposition des Beins ist, das als Stützbein der Zielperson dient, bis zu einem Endpunkt, der eine Position ist, die in einem ersten Abstand vor dem Startpunkt existiert, ist.

4. Informationsverarbeitungsvorrichtung (100) nach Anspruch 2, wobei
der vorbestimmte Bereich ein Bereich von einem Startpunkt, der eine Position ist, die in einem zweiten Abstand hinter der Landeposition des Beins existiert, das als Stützbein der Zielperson dient, bis zu einem Endpunkt, der eine Position ist, die in einem ersten Abstand vor dem Startpunkt liegt, ist.

5. Informationsverarbeitungsvorrichtung (100) nach Anspruch 4, wobei
für jede Kandidatengruppe die Steuereinheit (401) dazu ausgelegt ist, für jeden Schritt in der Gehperiode den zweiten Abstand gemäß einer Drehrichtung und einem Grad einer Drehung des Beins, das als Spielbein der Zielperson dient, einzustellen, und zu bestimmen, ob die Beziehung erscheint oder nicht.

6. Informationsverarbeitungsvorrichtung (100) nach einem der Ansprüche 2 bis 5, wobei
für jede Kandidatengruppe die Steuereinheit (401) dazu ausgelegt ist, den Bewertungswert der Kandidatengruppe zu berechnen, so dass der Bewertungswert der Kandidatengruppe erhöht wird, wenn die Differenz zwischen den kumulierten Wegstrecken beider Beine der Zielperson in der Gehperiode konstant ist.

7. Informationsverarbeitungsvorrichtung (100) nach einem der Ansprüche 1 bis 6, wobei

die Steuereinheit (401) ausgelegt ist zum
Erfassen von Messinformationen bezüglich der Bewegung einer Taille der Zielperson, und
Berechnen, für jede Kandidatengruppe, eines vorgegebenen Merkmalswerts für jeden Kandidaten basierend auf den Messinformationen bezüglich der Bewegung der Taille oder jedes Beines der Zielperson, und Auswählen einer der mehreren Kandidatengruppen basierend auf dem berechneten vorgegebenen Merkmalswert jedes Kandidaten.

8. Informationsverarbeitungsvorrichtung (100) nach einem der Ansprüche 1 bis 7, wobei

die Steuereinheit (401) ausgelegt ist zum
Erfassen von Messinformationen über die Reaktionskraft von dem Boden auf die Zielperson, und
Berechnen, für jede Kandidatengruppe, eines vorgegebenen Merkmalswerts für jeden Kandidaten basierend auf den Messinformationen bezüglich der Reaktionskraft und den Messinformationen bezüglich der Bewegung jedes Beines der Zielperson, und Auswählen einer der mehreren Kandidatengruppen basierend auf dem berechneten vorgegebenen Merkmalswerts jedes Kandidaten.

9. Informationsverarbeitungsvorrichtung (100) nach einem der Ansprüche 1 bis 8, wobei

die Steuereinheit (401) ausgelegt ist zum
Erfassen einer Wegstrecke der Zielperson, und
Auswählen, für jede Kandidatengruppe, einer der mehreren Kandidatengruppen basierend auf einer Differenz zwischen der kumulierten Wegstrecke des Beins, das in einem finalen Schritt der Gehperiode als Spielbein der Zielperson dient, und der Wegstrecke der Zielperson in der Gehperiode.

**10.** Informationsverarbeitungsvorrichtung (100) nach einem der Ansprüche 1 bis 9, wobei
als die Gehperiode eine Zeitspanne eingestellt wird, in der eine Gehbewegung durch die Zielperson das durchführt wird und die Anzahl der Schritte der Zielperson geringer als eine vorbestimmte Anzahl von Schritten ist.

**11.** Informationsverarbeitungsvorrichtung (100) nach einem der Ansprüche 1 bis 10, wobei

die Steuereinheit (401) ausgelegt ist zum
Spezifizieren der Schrittlänge der Zielperson für jeden Schritt in der Gehperiode basierend auf der ausgewählten der Kandidatengruppen, und
Ausgeben der spezifizierten Schrittlänge.

**12.** Informationsverarbeitungssystem (200), umfassend:

ein Messinstrument (201); und
eine Informationsverarbeitungsvorrichtung (100) nach einem der Ansprüche 1 bis 11, die mit dem Messinstrument (201) kommunizieren kann, wobei
das Messinstrument dazu ausgelegt ist, die Messinformationen zu generieren, und
die Informationsverarbeitungsvorrichtung (100) dazu ausgelegt ist, die Messinformationen von dem Messinstrument (201) zu erfassen.

**13.** Informationsverarbeitungsverfahren zum Bewirken, dass ein Computer die Verarbeitung auszuführen eines:

Erfassens von Messinformationen bezüglich einer Bewegung jedes Beins einer Zielperson;
Spezifizierens, für jeden Schritt in einer Gehperiode der Zielperson, eines oder mehrerer Kandidaten für einen Zeitpunkt, der einer Landung eines Beins entspricht, das als Spielbein der Zielperson dient, basierend auf den erfassten Messinformationen;
Spezifizierens mehrerer Kandidatengruppen, die durch Auswählen eines der einen oder mehreren für jeden Schritt in der Gehperiode spezifizierten Kandidaten erhalten werden, und Kombinieren der ausgewählten Kandidaten;
Berechnens, für jede Kandidatengruppe aus den mehreren spezifizierten Kandidatengruppen, einer Schrittlänge für jeden Schritt in der Gehperiode, und Berechnens, für jeden Schritt in der Gehperiode, einer kumulativen Wegstrecke des Beins, das als Spielbein der Zielperson dient, basierend auf der berechneten Schrittlänge jedes Schritts in der Gehperiode;
Bewertens, für jede Kandidatengruppe, eines Grades eines Erscheinens einer Beziehung basierend auf der kumulierten Wegstrecke, die für jeden Schritt in der Gehperiode berechnet wurde, wobei die Beziehung eine Beziehung ist, in der eine Landeposition des Beins, das als Spielbein der Zielperson dient, in einen vorbestimmten Bereich einer Landeposition eines Beins fällt, das während der Gehperiode als Stützbein der Zielperson dient;
Auswählens einer der mehreren Kandidatengruppen basierend auf einem Ergebnis der Bewertung; und
Ausgebens der ausgewählten der mehreren Kandidatengruppen.

## Revendications

**1.** Appareil de traitement d'informations (100) comprenant
une unité de commande (401) conçue pour :

acquérir des informations de mesure concernant le mouvement de chaque jambe d'un sujet ;
spécifier, pour chaque pas pendant une période de marche du sujet, un ou plusieurs candidats pour un moment correspondant à la réception d'une jambe ayant la fonction d'une jambe en mouvement du sujet sur la base des informations de mesure acquises ;
spécifier une pluralité de groupes candidats obtenus en sélectionnant l'un des un ou plusieurs candidats spécifiés pour chaque pas pendant la période de marche et en combinant les candidats sélectionnés ;
calculer, pour chaque groupe candidat de la pluralité de groupes candidats spécifiés, une longueur de pas de chaque pas pendant la période de marche et calculer, pour chaque pas pendant la période de marche, une distance de déplacement cumulative de la jambe ayant la fonction de jambe en mouvement du sujet sur la base de la longueur de pas calculée de chaque pas pendant la période de marche ;
évaluer, pour chaque groupe candidat, un degré d'apparition d'une relation sur la base de la distance de déplacement cumulative calculée pour chaque pas pendant la période de marche, la relation étant une relation dans laquelle une position de réception de la jambe ayant la fonction de jambe en mouvement du sujet se situe à l'intérieur d'une plage prédéterminée d'une position de réception d'une jambe ayant la fonction d'une jambe d'appui du sujet pendant la période de marche ;

sélectionner l'un de la pluralité de groupes candidats sur la base d'un résultat de l'évaluation ; et

délivrer en sortie le groupe sélectionné de la pluralité de groupes candidats.

2. Appareil de traitement d'informations (100) selon la revendication 1, dans lequel
l'unité de commande (401) est conçue pour
déterminer, pour chaque groupe candidat, si la relation apparaît ou non dans chaque pas de la période de marche et calculer une valeur d'évaluation du groupe candidat de sorte que la valeur d'évaluation du groupe candidat est améliorée lorsque le nombre de fois où la relation apparaît augmente, et
sélectionner l'un de la pluralité de groupes candidats sur la base de la valeur d'évaluation calculée de chaque groupe candidat.

3. Appareil de traitement d'informations (100) selon la revendication 2, dans lequel
la plage prédéterminée est une plage allant d'un point de départ qui est la position de réception de la jambe ayant la fonction de jambe d'appui du sujet à un point d'arrivée qui est une position existant à une première distance devant le point de départ.

4. Appareil de traitement d'informations (100) selon la revendication 2, dans lequel
la plage prédéterminée est une plage allant d'un point de départ qui est une position existant à une seconde distance derrière la position de réception de la jambe ayant la fonction de jambe d'appui du sujet à un point d'arrivée qui est une position existant à une première distance devant le point de départ.

5. Appareil de traitement d'informations (100) selon la revendication 4, dans lequel
pour chaque groupe candidat, l'unité de commande (401) est conçue pour définir, pour chaque pas pendant la période de marche, la seconde distance en fonction d'une direction de rotation et d'un degré de rotation de la jambe ayant la fonction de jambe en mouvement du sujet et déterminer si la relation apparaît ou non.

6. Appareil de traitement d'informations (100) selon l'une quelconque des revendications 2 à 5, dans lequel
pour chaque groupe candidat, l'unité de commande (401) est conçue pour calculer la valeur d'évaluation du groupe candidat de sorte que la valeur d'évaluation du groupe candidat est améliorée lorsqu'une différence entre les distances de déplacement cumulatives des deux jambes du sujet est constante pendant la période de marche.

7. Appareil de traitement d'informations (100) selon l'une quelconque des revendications 1 à 6, dans lequel
l'unité de commande (401) est conçue pour
acquérir des informations de mesure concernant le mouvement d'une taille du sujet, et
calculer, pour chaque groupe candidat, une valeur de caractéristique prédéterminée pour chaque candidat sur la base des informations de mesure concernant le mouvement de la taille ou de chaque jambe du sujet, et sélectionner l'un de la pluralité de groupes candidats sur la base de la valeur de caractéristique prédéterminée de chaque candidat.

8. Appareil de traitement d'informations (100) selon l'une quelconque des revendications 1 à 7, dans lequel
l'unité de commande (401) est conçue pour
acquérir des informations de mesure concernant la force de réaction depuis le sol vers le sujet, et
calculer, pour chaque groupe candidat, une valeur de caractéristique prédéterminée pour chaque candidat sur la base des informations de mesure concernant la force de réaction et les informations de mesure concernant le mouvement de chaque jambe du sujet, et sélectionner l'un de la pluralité de groupes candidats sur la base de la valeur de caractéristique prédéterminée calculée de chaque candidat.

9. Appareil de traitement d'informations (100) selon l'une quelconque des revendications 1 à 8, dans lequel
l'unité de commande (401) est conçue pour
acquérir une distance de déplacement du sujet, et
sélectionner, pour chaque groupe candidat, l'un de la pluralité de groupes candidats sur la base d'une différence entre la distance de déplacement cumulative de la jambe ayant la fonction de jambe en mouvement du sujet dans un pas final pendant la période de marche et la distance de déplacement du sujet pendant la période de marche.

10. Appareil de traitement d'informations (100) selon l'une quelconque des revendications 1 à 9, dans lequel
une période dans laquelle un mouvement de marche est effectué par le sujet et le nombre de pas du sujet est inférieur à un nombre prédéterminé de pas est définie en tant que période de marche.

11. Appareil de traitement d'informations (100) selon l'une quelconque des revendications 1 à 10, dans lequel l'unité de commande (401) est conçue pour

spécifier la longueur de pas du sujet pour chaque pas pendant la période de marche sur la base du groupe sélectionné parmi les groupes candidats, et

délivrer en sortie la longueur de pas spécifiée.

12. Système de traitement d'informations (200) comprenant :

un instrument de mesure (201) ; et

un appareil de traitement d'informations (100) selon l'une quelconque des revendications 1 à 11 qui peut communiquer avec l'instrument de mesure (201), dans lequel

l'instrument de mesure est conçu pour générer les informations de mesure, et

l'appareil de traitement d'informations (100) est conçu pour acquérir les informations de mesure à partir de l'instrument de mesure (201).

13. Procédé de traitement d'informations destiné à amener un ordinateur à exécuter le traitement :

d'acquisition d'informations de mesure concernant le mouvement de chaque jambe d'un sujet ;

de spécification, pour chaque pas pendant une période de marche du sujet, d'un ou plusieurs candidats pour un moment correspondant à la réception d'une jambe ayant la fonction d'une jambe en mouvement du sujet sur la base des informations de mesure acquises ;

de spécification d'une pluralité de groupes candidats obtenus en sélectionnant l'un des un ou plusieurs candidats spécifiés pour chaque pas pendant la période de marche et en combinant les candidats sélectionnés ;

de calcul, pour chaque groupe candidat de la pluralité de groupes de candidats spécifiés, d'une longueur de pas de chaque pas pendant la période de marche et le calcul, pour chaque pas pendant la période de marche, d'une distance de déplacement cumulative de la jambe ayant la fonction de jambe en mouvement du sujet sur la base de la longueur de pas calculée de chaque pas pendant la période de marche ;

d'évaluation, pour chaque groupe candidat, d'un degré d'apparition d'une relation sur la base de la distance de déplacement cumulative calculée pour chaque pas pendant la période de marche, la relation étant une relation dans laquelle une position de réception de la jambe ayant la fonction de jambe en mouvement du sujet se situe à l'intérieur d'une plage prédéterminée d'une position de réception d'une jambe ayant la fonction de jambe d'appui du sujet pendant la période de marche ;

la sélection d'un de la pluralité de groupes candidats sur la base d'un résultat de l'évaluation ; et

la délivrance en sortie du groupe sélectionné de la pluralité de groupes candidats.

# FIG. 1

# FIG. 2

# FIG. 3

# FIG. 4

# FIG. 5

EP 3 649 939 B1

FIG. 6

[SELECTION OF CANDIDATE GROUP]

MONOTONIC INCREASE

○ OK

| | SECOND STEP | FOURTH STEP |
LEFT LEG
RIGHT LEG
FIRST STEP — THIRD STEP

CUMULATIVE TRAVEL DISTANCE — NUMBER OF STEPS — 1 2 3 4

600

[CANDIDATES FOR heel-strike POINT]

[CANDIDATE GROUP]

CANDIDATE P1,P2      CANDIDATE R1,R2

LEFT LEG

RIGHT LEG

12:50:36    12:50:37    12:50:38

ACCELERATION   CANDIDATE Q

CANDIDATE S

| | FIRST STEP (LEFT) | SECOND STEP (RIGHT) | THIRD STEP (LEFT) | FOURTH STEP (RIGHT) |
|---|---|---|---|---|
| CANDIDATE GROUP A | P1 | Q | R1 | S |
| CANDIDATE GROUP B | P1 | Q | R2 | S |
| CANDIDATE GROUP C | P2 | Q | R1 | S |
| CANDIDATE GROUP A | P2 | Q | R2 | S |

✕ NG

| | SECOND STEP | FOURTH STEP |
LEFT LEG
RIGHT LEG
FIRST STEP — THIRD STEP

CUMULATIVE TRAVEL DISTANCE — NUMBER OF STEPS — 1 2 3 4

EP 3 649 939 B1

# FIG. 7

201

201

RIGHT LEG

LEFT LEG

201

SENSOR DEVICE

# FIG. 8

SAGITTAL PLANE

TRAVERSE PLANE

ANGULAR VELOCITY
ON TRAVERSE PLANE

ANGULAR VELOCITY
ON SAGITTAL PLANE

# FIG. 9

VERTICAL DIRECTION

LATERAL DIRECTION

LONGITUDINAL DIRECTION

# FIG. 10

# FIG. 11

ANGULAR VELOCITY

DETECTED WALKING PERIOD

TIME

1100

| PERIOD NUMBER | START TIME | END TIME | NUMBER OF STEPS |
|---|---|---|---|
| 1 | 02:00:04 | 02:08:32 | 256 |
| 2 | 02:41:01 | 02:41:30 | 45 |
| 3 | 02:43:35 | 02:43:45 | 13 |
| 4 | 02:46:55 | 02:47:51 | 85 |
| 5 | 02:48:05 | 02:49:49 | 132 |

EP 3 649 939 B1

EP 3 649 939 B1

# FIG. 12

# FIG. 13

[CANDIDATES FOR heel-strike POINT]

# FIG. 14

[CANDIDATES FOR heel-strike POINT]

[CREATION OF CANDIDATE GROUP]

1400

| CANDIDATE GROUP | FIRST STEP (LEFT) | SECOND STEP (RIGHT) | THIRD STEP (LEFT) | FOURTH STEP (RIGHT) |
|---|---|---|---|---|
| CANDIDATE GROUP A | P1 | Q | R1 | S |
| CANDIDATE GROUP B | P1 | Q | R2 | S |
| CANDIDATE GROUP C | P2 | Q | R1 | S |
| CANDIDATE GROUP D | P2 | Q | R2 | S |

# FIG. 15

EP 3 649 939 B1

12:19:29             12:19:30

T1           T2

Distance traveled SL

zero work    zero knee torque

θ

θ

Leg length L

Distance traveled SL

LENGTH OF LEG

L

RIGHT LEG PERFORMS heel-strike

LEFT LEG PERFORMS heel-strike

θ

# FIG. 16

[CANDIDATE GROUP]

| CANDIDATE GROUP | FIRST STEP (LEFT LEG) | SECOND STEP (RIGHT LEG) | THIRD STEP (LEFT LEG) | FOURTH STEP (RIGHT LEG) |
|---|---|---|---|---|
| CANDIDATE GROUP A | P1 | Q | R1 | S |
| CANDIDATE GROUP B | P1 | Q | R2 | S |
| CANDIDATE GROUP C | P2 | Q | R1 | S |
| CANDIDATE GROUP D | P2 | Q | R2 | S |

[STEP LENGTH CALCULATION RESULT]

1600

| CANDIDATE GROUP | FIRST STEP (LEFT LEG) | | SECOND STEP (RIGHT LEG) | | THIRD STEP (LEFT LEG) | | FOURTH STEP (RIGHT LEG) | |
|---|---|---|---|---|---|---|---|---|
| CANDIDATE GROUP A | P1 | 27cm | Q | 73cm | R1 | 67cm | S | 65cm |
| CANDIDATE GROUP B | P1 | 27cm | Q | 73cm | R2 | 39cm | S | 80cm |
| CANDIDATE GROUP C | P2 | 20cm | Q | 68cm | R1 | 67cm | S | 65cm |
| CANDIDATE GROUP D | P2 | 20cm | Q | 68cm | R2 | 39cm | S | 80cm |

# FIG. 17

[STEP LENGTH CALCULATION RESULT]

| CANDIDATE GROUP | FIRST STEP (LEFT LEG) | | SECOND STEP (RIGHT LEG) | | THIRD STEP (LEFT LEG) | | FOURTH STEP (RIGHT LEG) | |
|---|---|---|---|---|---|---|---|---|
| CANDIDATE GROUP A | P1 | 27cm | Q | 73cm | R1 | 67cm | S | 65cm |
| CANDIDATE GROUP B | P1 | 27cm | Q | 73cm | R2 | 39cm | S | 80cm |
| CANDIDATE GROUP C | P2 | 20cm | Q | 68cm | R1 | 67cm | S | 65cm |
| CANDIDATE GROUP D | P2 | 20cm | Q | 68cm | R2 | 39cm | S | 80cm |

[CUMULATIVE TRAVEL DISTANCE CALCULATION RESULT]  1700

| CANDIDATE GROUP | FIRST STEP (LEFT LEG) | | SECOND STEP (RIGHT LEG) | | THIRD STEP (LEFT LEG) | | FOURTH STEP (RIGHT LEG) | |
|---|---|---|---|---|---|---|---|---|
| CANDIDATE GROUP A | P1 | 27cm | Q | 73cm | R1 | 94cm | S | 138cm |
| CANDIDATE GROUP B | P1 | 27cm | Q | 73cm | R2 | 66cm | S | 153cm |
| CANDIDATE GROUP C | P2 | 20cm | Q | 68cm | R1 | 87cm | S | 133cm |
| CANDIDATE GROUP D | P2 | 20cm | Q | 68cm | R2 | 59cm | S | 148cm |

LEFT LEG — FIRST STEP, THIRD STEP

RIGHT LEG — SECOND STEP, FOURTH STEP

STEP LENGTH OF FIRST STEP

STEP LENGTH OF THIRD STEP

CUMULATIVE TRAVEL DISTANCE IN THIRD STEP

CUMULATIVE TRAVEL DISTANCE IN FIRST STEP

EP 3 649 939 B1

# FIG. 18

[CERTAINTY FACTOR CALCULATION RESULT] 1800

| CANDIDATE GROUP | FIRST STEP (LEFT LEG) | SECOND STEP (RIGHT LEG) | THIRD STEP (LEFT LEG) | FOURTH STEP (RIGHT LEG) | FIRST CERTAINTY FACTOR |
|---|---|---|---|---|---|
| CANDIDATE GROUP A | P1 | Q | R1 | S | 10.2 |
| CANDIDATE GROUP B | P1 | Q | R2 | S | 0 |
| CANDIDATE GROUP C | P2 | Q | R1 | S | 3.45 |
| CANDIDATE GROUP D | P2 | Q | R2 | S | 0 |

# FIG. 19

# FIG. 20

H(dₙ): EVALUATION VALUE

2000

# FIG. 21

# FIG. 22

H(d_n): EVALUATION VALUE

2200

1

1

0

ε     0     d_n: DIFFERENCE BETWEEN
            CUMULATIVE TRAVEL DISTANCES

# FIG. 23

O.K

SECOND STEP    FOURTH STEP    SIXTH STEP

LEFT LEG

RIGHT LEG

FIRST STEP    THIRD STEP    FIFTH STEP

VARIATION IS SMALL

N.G

SECOND STEP    FOURTH STEP    SIXTH STEP

LEFT LEG

RIGHT LEG

FIRST STEP    THIRD STEP    FIFTH STEP

VARIATION IS LARGE

# FIG. 24

# FIG. 25

# FIG. 26

# FIG. 27

# FIG. 28

2800

| CANDIDATE GROUP | FIRST STEP (LEFT LEG) | | | | | | | ... | FOURTH STEP (RIGHT LEG) | | | | | | |
|---|---|---|---|---|---|---|---|---|---|---|---|---|---|---|---|
| CANDIDATE GROUP A | P1 | 1.3 | 0.2 | 5.5 | 2.1 | -56 | 2.5 | ... | S | 1.2 | 0.3 | 4.3 | 1.8 | -39 | 1.5 |
| CANDIDATE GROUP B | P1 | 1.3 | 0.2 | 5.5 | 2.1 | -56 | 2.5 | ... | S | 1.2 | 0.3 | 4.3 | 1.8 | -39 | 1.5 |
| CANDIDATE GROUP C | P2 | 0.4 | 0.1 | 3.2 | 0.1 | -46 | 2.3 | ... | S | 1.2 | 0.3 | 4.3 | 1.8 | -39 | 1.5 |
| CANDIDATE GROUP D | P2 | 0.4 | 0.1 | 3.2 | 0.1 | -46 | 2.3 | ... | S | 1.2 | 0.3 | 4.3 | 1.8 | -39 | 1.5 |
| | CANDIDATE | $\alpha_1$ | $\alpha_2$ | $\alpha_3$ | $\alpha_4$ | $\beta_1$ | $\beta_2$ | | CANDIDATE | $\alpha_1$ | $\alpha_2$ | $\alpha_3$ | $\alpha_4$ | $\beta_1$ | $\beta_2$ |

# FIG. 29

EP 3 649 939 B1

| CANDIDATE GROUP | FIRST STEP (LEFT LEG) | | | | $\cdots$ | FOURTH STEP (RIGHT LEG) | | | |
|---|---|---|---|---|---|---|---|---|---|
| CANDIDATE GROUP A | 1.3 | 0.2 | 5.5 | 2.1 | $\cdots$ | 1.2 | 0.3 | 4.3 | 1.8 |
| CANDIDATE GROUP B | 1.3 | 0.2 | 5.5 | 2.1 | $\cdots$ | 1.2 | 0.3 | 4.3 | 1.8 |
| CANDIDATE GROUP C | 0.4 | 0.1 | 3.2 | 0.1 | $\cdots$ | 1.2 | 0.3 | 4.3 | 1.8 |
| CANDIDATE GROUP D | 0.4 | 0.1 | 3.2 | 0.1 | $\cdots$ | 1.2 | 0.3 | 4.3 | 1.8 |
| | $\alpha_1$ | $\alpha_2$ | $\alpha_3$ | $\alpha_4$ | | | | | |

2900

| VARIATION OF $\beta_1$ | VARIATION OF $\beta_2$ |
|---|---|
| 8.4 | 17.6 |
| 5.3 | 21.2 |
| 2.6 | 16.0 |
| 1.2 | 12.3 |

# FIG. 30

$H(\alpha_i^n)$: EVALUATION VALUE     3000

1

0

$th_i$

$\alpha_i^n$: FEATURE VALUE OF MOVEMENT

# FIG. 31

3100

| CANDIDATE GROUP | FIRST STEP (LEFT LEG) | SECOND STEP (RIGHT LEG) | THIRD STEP (LEFT LEG) | FOURTH STEP (RIGHT LEG) | FIRST CERTAINTY FACTOR | SECOND CERTAINTY FACTOR | FINAL CERTAINTY FACTOR (PRODUCT OF FIRST CERTAINTY FACTOR AND SECOND CERTAINTY FACTOR) |
|---|---|---|---|---|---|---|---|
| CANDIDATE GROUP A | P1 | Q | R1 | S | 10.2 | 1 | 10.2 |
| CANDIDATE GROUP B | P1 | Q | R2 | S | 0 | 1 | 0 |
| CANDIDATE GROUP C | P2 | Q | R1 | S | 3.45 | 0 | 0 |
| CANDIDATE GROUP D | P2 | Q | R2 | S | 0 | 0 | 0 |

| CANDIDATE GROUP | FIRST STEP (LEFT LEG) | SECOND STEP (RIGHT LEG) | THIRD STEP (LEFT LEG) | FOURTH STEP (RIGHT LEG) |
|---|---|---|---|---|
| STEP LENGTH | 27cm | 73cm | 67cm | 65cm |

3101

EP 3 649 939 B1

# FIG. 32

3200

CANDIDATE FOR heel-strike POINT: T

LENGTH OF PERIOD: v | LENGTH OF PERIOD: v

γ2

LONGITUDINAL ACCELERATION OF WAIST

TIME

γ1

# FIG. 33

EP 3 649 939 B1

REACTION FORCE
SENSOR DEVICE

heel-strike

FLOOR REACTION FORCE

heel-strike POINT

TIME

MOVEMENT FEATURE X

MOVEMENT FEATURE Y

MOVEMENT FEATURE
OF CANDIDATE GROUP

LEARNED MOVEMENT FEATURE

# FIG. 34

CUMULATIVE TRAVEL DISTANCES IN FINAL STEP

3400

CANDIDATE GROUP A   CANDIDATE GROUP B   CANDIDATE GROUP C   CANDIDATE GROUP D

TRAVEL DISTANCE CALCULATED BY POSITIONING MEANS

# FIG. 35

# FIG. 36

EP 3 649 939 B1

# FIG. 37A

START

ACQUIRE SENSOR DATA FOR CERTAIN PERIOD — S3701

SPECIFY ONE OR MORE WALKING PERIODS — S3702

C → SELECT ONE OF ONE OR MORE WALKING PERIODS — S3703

DIVIDE WALKING PERIOD INTO ONE OR MORE PARTIAL PERIODS — S3704

B → SELECT ONE OF ONE OR MORE PARTIAL PERIODS — S3705

SPECIFY CANDIDATES FOR HEEL-STRIKE POINT — S3706

SPECIFY PLURALITY OF CANDIDATE GROUPS — S3707

SELECT ONE OF PLURALITY OF CANDIDATE GROUPS — S3708

EXECUTE FINAL CERTAINTY FACTOR CALCULATION PROCESSING — S3709

ALL OF PLURALITY OF CANDIDATE GROUPS HAVE BEEN SELECTED? — S3710

NO

YES

A

# FIG. 37B

(A)

SELECT CANDIDATE GROUP ON BASIS OF FINAL CERTAINTY FACTOR — S3711

OUTPUT STEP LENGTH OF SUBJECT ON BASIS OF SELECTED CANDIDATE GROUP — S3712

(B) — NO — ALL OF ONE OR MORE PARTIAL PERIODS HAVE BEEN SELECTED? — S3713

YES

(C) — NO — ALL OF ONE OR MORE WALKING PERIODS HAVE BEEN SELECTED? — S3714

YES

END

# FIG. 38

START

S3801 — CALCULATE STEP LENGTH FOR EACH STEP OF SELECTED CANDIDATE GROUP

S3802 — CALCULATE CUMULATIVE TRAVEL DISTANCE IN EACH STEP OF SELECTED CANDIDATE GROUP

S3803 — EXECUTE FIRST CERTAINTY FACTOR CALCULATION PROCESSING

S3804 — CALCULATE FEATURE VALUES OF LEFT LEG AND RIGHT LEG

S3805 — THERE IS ACCELERATION DATA OF LONGITUDINAL ACCELERATION OF WAIST? —NO

YES

S3806 — CALCULATE FEATURE VALUE OF WAIST

S3807 — CALCULATE SECOND CERTAINTY FACTOR

S3808 — THERE IS GPS DATA? —NO

YES

S3809 — CALCULATE THIRD CERTAINTY FACTOR

S3810 — THERE IS REACTION FORCE DATA? —NO

YES

S3811 — CALCULATE SECOND CERTAINTY FACTOR AGAIN

S3812 — CALCULATE FINAL CERTAINTY FACTOR

END

# FIG. 39

```
                    ┌──────────────┐
                    │    START     │
                    └──────────────┘
                           │
                           ▼
S3901 ┌─────────────────────────────────────────────┐
      │         READ PARAMETER VALUE ε               │
      └─────────────────────────────────────────────┘
                           │
                           ▼
S3902 ┌─────────────────────────────────────────────┐
      │        CALCULATE ADJUSTMENT VALUE δ          │
      └─────────────────────────────────────────────┘
                           │
                           ▼
S3903 ┌─────────────────────────────────────────────┐
      │  CALCULATE DEGREE OF VARIATION V OF DIFFERENCE│
      │              BETWEEN DISTANCES                │
      └─────────────────────────────────────────────┘
                           │
                           ▼
S3904 ┌─────────────────────────────────────────────┐
      │         CALCULATE VALUE OF EVALUATION         │
      │            FUNCTION E(ε, δ, V)                │
      └─────────────────────────────────────────────┘
                           │
                           ▼
                    ┌──────────────┐
                    │     END      │
                    └──────────────┘
```

## REFERENCES CITED IN THE DESCRIPTION

**Patent documents cited in the description**

- JP 2004163168 A
- JP 2008175559 A
- JP 2012179114 A
- JP 2002197437 A
- US 2016100776 A1

**Non-patent literature cited in the description**

- A robust step length estimation system for human gait using motion sensors. **WU, XIAOXU ; YAN WANG ; GREGORY POTTIE.** Proceedings of the conference on Wireless Health. ACM, 2015
- **BAGALCIAGUE F et al.** Unstable gait assessment with a portable analysis system. *2014 IEEE INTERNATIONAL INSTRUMENTATION AND MEASUREMENT TECHNOLOGY CONFERENCE (I2MTC) PROCEEDINGS,* 12 May 2014, 181-185